(19) Europäisches Patentamt
European Patent Office
Office européen des brevets

(11) EP 1 206 446 B1

(12) EUROPÄISCHE PATENTSCHRIFT

(45) Veröffentlichungstag und Bekanntmachung des Hinweises auf die Patenterteilung:
**26.05.2004 Patentblatt 2004/22**

(21) Anmeldenummer: **00956375.0**

(22) Anmeldetag: **02.08.2000**

(51) Int Cl.[7]: **C07C 257/18**, C07D 295/18, C07D 207/12, A61K 31/40, A61K 31/4164, A61P 7/02, C07D 295/12, C07C 311/21, C07C 311/46, C07D 233/54

(86) Internationale Anmeldenummer:
**PCT/EP2000/007457**

(87) Internationale Veröffentlichungsnummer:
**WO 2001/010823 (15.02.2001 Gazette 2001/07)**

(54) **CARBONSÄUREAMIDE, DEREN HERSTELLUNG UND DEREN VERWENDUNG ALS ARZNEIMITTEL**

CARBOXYLIC ACID AMIDES, THEIR PRODUCTION AND THEIR USE AS DRUGS

AMIDES D'ACIDE CARBOXYLIQUE, LEUR PRODUCTION ET LEUR UTILISATION COMME MEDICAMENTS

(84) Benannte Vertragsstaaten:
**AT BE CH CY DE DK ES FI FR GB GR IE IT LI LU MC NL PT SE**

(30) Priorität: **07.08.1999 DE 19937494**
**24.05.2000 DE 10025663**

(43) Veröffentlichungstag der Anmeldung:
**22.05.2002 Patentblatt 2002/21**

(73) Patentinhaber: **Boehringer Ingelheim Pharma GmbH & Co.KG**
**55218 Ingelheim am Rhein (DE)**

(72) Erfinder:
- **RIES, Uwe**
  **D-88400 Biberach (DE)**
- **PRIEPKE, Henning**
  **D-88447 Warthausen (DE)**
- **HECKEL, Armin**
  **D-88400 Biberach (DE)**
- **NAR, Herbert**
  **D-88441 Mittelbiberach (DE)**
- **WIENEN, Wolfgang**
  **D-88400 Biberach (DE)**
- **STASSEN, Jean, Marie**
  **B-3012 Leuven (BE)**

(56) Entgegenhaltungen:
**GB-A- 2 007 663** **US-A- 5 726 159**

- **JUN SAKAGUCHI ET AL.: "Synthesis, Gastrointestinal Prokinetic Activity and Structure-Activity Relationships of Novel N-[[2-(Dialkylamino)ethoxy]benzyl]- benzamide Derivatives" CHEMICAL AND PHARMACEUTICAL BULLETIN, Bd. 40, Nr. 1, 1992, Seiten 202-211, XP002152593 TOKYO JP**
- **L. SIMON ET AL.: "Darstellung von substituierten Isochinolinderivaten" PHARMAZIE., Bd. 29, Nr. 5, 1974, Seiten 313-314, XP002152594 BERLIN DD**
- **D. LABES ET AL.: "Free-Wilson-Analyse der Hemmwirkung von 4-substituierten Benzamidinen gegenüber Thrombin, Plasmin und Trypsin" PHARMAZIE., Bd. 34, Nr. 9, 1979, Seiten 554-555, XP002152595 BERLIN DD**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Gegenstand der vorliegenden Erfindung sind Carbonsäureamide der allgemeinen Formel

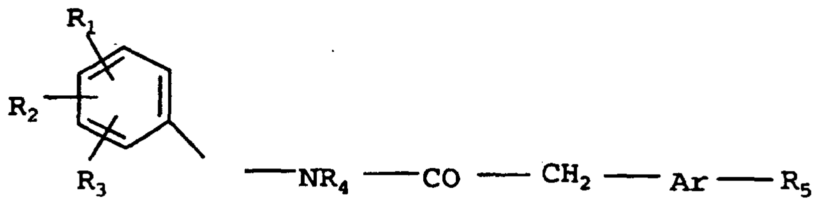

(I),

deren Tautomere, deren Stereoisomere, deren Gemische, deren Prodrugs, deren Derivate, die an Stelle einer Carboxygruppe eine unter physiologischen Bedingungen negativ geladene Gruppe enthalten, und deren Salze, insbesondere deren physiologisch verträglichen Salze mit anorganischen oder organischen Säuren oder Basen, welche wertvolle Eigenschaften aufweisen.

Die Verbindungen der obigen allgemeinen Formel I, in denen $R_5$ eine Cyanogruppe darstellt, stellen wertvolle Zwischenprodukte zur Herstellung der übrigen Verbindungen der allgemeinen Formel I dar, und die Verbindungen der obigen allgemeinen Formel I, in denen $R_5$ eine der nachfolgenden Amidinogruppen darstellt, sowie deren Tautomere, deren Stereoisomere, deren Gemische, deren Prodrugs, deren Derivate, die an Stelle einer Carboxygruppe eine unter physiologischen Bedingungen negativ geladene Gruppe enthalten, und deren Salze, insbesondere deren physiologisch verträgliche Salze mit anorganischen oder organischen Salze, und deren Stereoisomere weisen wertvolle pharmakologische Eigenschaften auf, insbesondere eine antithrombotische Wirkung und eine Faktor Xa-inhibierende Wirkung.

"Im US Patent 5,726,159 werden bereits Thrombinhemmer beschrieben und die britische Patentanmeldung GB 2 007 663 A offenbart sulfonylierte $\bar{\omega}$-Amidinophenyl-$\alpha$-aminocarbonsäure-amide mit antikoagulatorischer Aktivität. Weiterhin beschreiben D. Labes und V. Hagen (Pharmazie, Band 34, Nr. 9, 1979, Seiten 554-555, Berlin: *Free-Wilson-Analyse der Hemmwirkung von 4-substituierten Benzamidinen gegenüber Thrombin, Plasmin und Trypsin*) die Beziehungen zwischen Struktur und Hemmwirkung anhand von 4-Amidinophenylverbindungen."

Gegenstand der vorliegenden Anmeldung sind somit die neuen Verbindungen der obigen allgemeinen Formel I sowie deren Herstellung, die die pharmakologisch wirksamen Verbindungen enthaltende Arzneimittel, deren Herstellung und Verwendung.

In der obigen allgemeinen Formel bedeutet

Ar eine gegebenenfalls durch ein Fluor-, Chlor- oder Bromatom, durch eine Trifluormethyl-, $C_{1-3}$-Alkyl-, Hydroxy-, $C_{1-3}$-Alkoxy-, Phenyl-$C_{1-3}$-alkoxy-, Amino-, $C_{1-3}$-Alkylamino- oder Di-($C_{1-3}$-Alkyl)-aminogruppe substituierte Phenylen- oder Naphthylengruppe, wobei die Phenylengruppe durch ein weiteres Fluor-, Chloroder Bromatom oder durch eine weitere $C_{1-3}$-Alkylgruppe substituiert sein kann,

eine Thienylen-, Thiazolylen-, Pyridinylen-, Pyrimidinylen-, Pyrazinylen- oder Pyridazinylengruppe, die im Kohlenstoffgerüst gegebenenfalls durch eine $C_{1-3}$-Alkylgruppe substituiert ist,

$R_1$ eine gegebenenfalls durch eine Amino-, $C_{1-3}$-Alkylamino-, Di-($C_{1-3}$-Alkyl)-amino-, Phenyl-, Naphthyl-, Heteroaryl- oder 4- bis 7-gliedrige Cycloalkyleniminogruppe substituierte $C_{1-3}$-Alkylgruppe,

eine $C_{3-7}$-Cycloalkylgruppe, die in 1-Stellung durch eine 5- bis 7-gliedrige Cycloalkyleniminocarbonylgruppe substituiert ist,

eine Amino-, $C_{1-5}$-Alkylamino-, $C_{5-7}$-Cycloalkylamino- oder Phenyl-$C_{1-3}$-alkylaminogruppe, die jeweils am Aminstickstoffatom durch eine Benzoyl- oder Phenylsulfonylgruppe oder durch eine gegebenenfalls im $C_{1-3}$-Alkylteil durch eine Carboxygruppe substituierte $C_{1-3}$-Alkyl- oder $C_{1-3}$-Alkylcarbonylgruppe substituiert sein kann,

eine gegebenenfalls durch eine $C_{1-3}$-Alkylgruppe substituierte 4-bis 7-gliedrige Cycloalkyleniminocarbonyl- oder Cycloalkyleniminosulfonylgruppe,

eine gegebenenfalls durch eine oder zwei $C_{1-3}$-Alkylgruppen substituierte Aminosulfonylgruppe,

eine gegebenenfalls durch ein Fluor-, Chlor- oder Bromatom, durch eine Trifluormethyl-, Aminosulfonyl-, $C_{1-3}$-Alkyl- oder $C_{1-3}$-Alkoxygruppe substituierte Phenylgruppe, die zusätzlich durch ein Fluor-, Chlor- oder Bromatom, durch eine Trifluormethyl-, $C_{1-3}$-Alkyl- oder $C_{1-3}$-Alkoxygruppe substituiert sein kann,

eine $C_{1-3}$-Alkoxy-, Phenyl-$C_{1-3}$-alkoxy-, Heteroaryloxy- oder Heteroaryloxy-$C_{1-3}$-alkoxygruppe, in der der Alkoxyteil jeweils in 2- oder 3-Stellung auch durch eine Amino-, $C_{1-3}$-Alkylamino- oder Di-($C_{1-3}$-Alkyl)-aminogruppe substituiert sein kann,

eine $C_{3-7}$-Cycloalkoxygruppe, wobei die Methylengruppe in 3- oder 4-Stellung in einer $C_{5-7}$-Cycloalkoxygruppe durch eine -NH-Gruppe ersetzt sein kann, wobei die -NH-Gruppe

durch eine $C_{1-3}$-Alkylgruppe, die in 2- oder 3-Stellung durch eine Amino-, $C_{1-3}$-Alkylamino- oder Di-($C_{1-3}$-Alkyl)-aminogruppe substituiert sein kann, durch eine $C_{1-3}$-Alkylcarbonyl-, Arylcarbonyl- oder Arylsulfonylgruppe oder

durch Aminocarbonyl-, $C_{1-3}$-Alkylaminocarbonyl- oder Di-($C_{1-3}$-Alkyl)-aminocarbonylgruppe, in denen jeweils das Sauerstoffatom der Carbonylgruppe durch eine Iminogruppe ersetzt ist, substituiert sein kann,

$R_2$ ein Wasserstoff-, Fluor-, Chlor- oder Bromatom, eine $C_{1-3}$-Alkyl-, Hydroxy- oder $C_{1-3}$-Alkoxygruppe,

$R_3$ ein Wasserstoffatom oder eine $C_{1-3}$-Alkylgruppe,

$R_4$ ein Wasserstoffatom oder eine gegebenenfalls durch eine Carboxygruppe substituierte $C_{1-3}$-Alkylgruppe und

$R_5$ eine Cyanogruppe oder eine gegebenenfalls durch eine oder zwei $C_{1-3}$-Alkylgruppen substituierte Amidinogruppe,

wobei unter den vorstehend erwähnten Heteroarylgruppen eine gegebenenfalls durch eine $C_{1-3}$-Alkylgruppe substituierte 5-gliedrige Heteroarylgruppe, die im heteroaromatischen Teil

eine gegebenenfalls durch eine $C_{1-3}$-Alkylgruppe substituierte Iminogruppe, ein Sauerstoff- oder Schwefelatom,

eine gegebenenfalls durch eine $C_{1-3}$-Alkylgruppe substituierte Iminogruppe und ein Sauerstoff-, Schwefel- oder Stickstoffatom,

eine gegebenenfalls durch eine $C_{1-3}$-Alkylgruppe substituierte Iminogruppe und zwei Stickstoffatome oder

ein Sauerstoff- oder Schwefelatom und zwei Stickstoffatome enthält,

zu verstehen ist,

die bei der Definition der vorstehend erwähnten Resten erwähnten Carboxygruppen durch eine in-vivo in eine Carboxygruppe überführbare Gruppe oder durch eine unter physiologischen Bedingungen negativ geladene Gruppe ersetzt sein und

die bei der Definition der vorstehend erwähnten Resten erwähnten Amino- und Iminogruppen durch einen in vivo abspaltbaren Rest substituiert sein können, wobei

unter einer in-vivo in eine Carboxygruppe überführbaren Gruppe beispielsweise eine Hydroxmethylgruppe, eine mit einem Alkohol veresterte Carboxygruppe, in der der alkoholische Teil vorzugsweise ein $C_{1-6}$-Alkanol, ein Phenyl-$C_{1-3}$-alkanol, ein $C_{3-9}$-Cycloalkanol, wobei ein $C_{5-8}$-Cycloalkanol zusätzlich durch eine oder zwei $C_{1-3}$-Alkylgruppen substituiert sein kann, ein $C_{5-8}$-Cycloalkanol, in dem eine Methylengruppe in 3- oder 4-Stellung durch ein Sauerstoffatom oder durch eine gegebenenfalls durch eine $C_{1-3}$-Alkyl-, Phenyl-$C_{1-3}$-alkyl-, Phenyl-$C_{1-3}$-alkoxycarbonyl- oder $C_{2-6}$-Alkanoylgruppe substituierte Iminogruppe ersetzt ist und der Cycloalkanolteil zusätzlich durch eine oder zwei $C_{1-3}$-Alkylgruppen substituiert sein kann, ein $C_{4-7}$-Cycloalkenol, ein $C_{3-5}$-Alkenol, ein Phenyl-$C_{3-5}$-alkenol, ein $C_{3-5}$-Alkinol oder Phenyl-$C_{3-5}$-alkinol mit der Maßgabe, daß keine Bindung an das Sauerstoffatom von einem Kohlenstoffatom ausgeht, welches eine Doppel- oder Dreifachbindung trägt, ein $C_{3-8}$-Cycloalkyl-$C_{1-3}$-alkanol, ein Bicycloalkanol mit insgesamt 8 bis 10 Kohlenstoffatomen, das im Bicycloalkylteil zusätzlich durch eine oder zwei $C_{1-3}$-Alkylgruppen substituiert sein kann, ein 1,3-Dihydro-3-oxo-1-isobenzfuranol oder ein Alkohol der Formel

$$R_a\text{-CO-O-}(R_bCR_c)\text{-OH},$$

verstanden wird, in dem

$R_a$ eine $C_{1-8}$-Alkyl-, $C_{5-7}$-Cycloalkyl-, Phenyl- oder Phenyl-$C_{1-3}$-alkylgruppe,

$R_b$ ein Wasserstoffatom, eine $C_{1-3}$-Alkyl-, $C_{5-7}$-Cycloalkyl- oder Phenylgruppe und

$R_c$ ein Wasserstoffatom oder eine $C_{1-3}$-Alkylgruppe darstellen,

unter einer unter physiologischen Bedingungen negativ geladenen Gruppe eine Tetrazol-5-yl-, Phenylcarbonylaminocarbonyl-, Trifluormethylcarbonylaminocarbonyl-, $C_{1-6}$-Alkylsulfonylamino-, Phenylsulfonylamino-, Benzylsulfonylamino-, Trifluormethylsulfonylamino-, $C_{1-6}$-Alkylsulfonylaminocarbonyl-, Phenylsulfonylaminocarbonyl-, Benzylsulfonylaminocarbonyl- oder Perfluor-$C_{1-6}$-alkylsulfonylaminocarbonylgruppe

und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Brom- oder Jodatome, durch $C_{1-3}$-Alkyl- oder $C_{1-3}$-Alkoxygruppen mono- oder disubstituierte Benzoylgruppe, wobei die Substituenten gleich oder verschieden sein können, eine Pyridinoylgruppe oder eine $C_{1-16}$-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine $C_{1-16}$-Alkoxycarbonyl- oder $C_{1-16}$-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert. Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy- oder Hexadecylcarbonyloxygruppe, eine Phenyl-$C_{1-6}$-alkoxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch $C_{1-6}$-Alkyl- oder $C_{1-7}$-Cycloalkylgruppen mono- oder disubstituiert und die Substituenten

gleich oder verschieden sein können, eine $C_{1-3}$-Alkylsulfonyl-$C_{2-4}$-alkoxycarbonyl-, $C_{1-3}$-Alkoxy-$C_{2-4}$-alkoxy-$C_{2-4}$-alkoxycarbonyl-, $R_a$-CO-O-($R_bCR_c$)-O-CO-, $C_{1-6}$-Alkyl-CO-NH-($R_dCR_e$)-O-CO- oder $C_{1-6}$-Alkyl-CO-O-($R_dCR_e$)-($R_dCR_e$)-O-CO-Gruppe, in denen $R_a$ bis $R_c$ wie vorstehend erwähnt definiert sind und

$R_d$ und $R_e$, die gleich oder verschieden sein können, Wasserstoffatome oder $C_{1-3}$-Alkylgruppen darstellen, verstanden werden,

deren Isomere und deren Salze.

insbesondere jedoch, wenn m, n, Ar und $R_2$ bis $R_5$ wie vorstehend erwähnt definiert sind, bedeutet

$R_1$ eine gegebenenfalls durch eine Amino-, $C_{1-3}$-Alkylamino-, Di-($C_{1-3}$-Alkyl)-amino-, Phenyl-, Naphthyl- oder Heteroarylgruppe substituierte $C_{1-3}$-Alkylgruppe,

eine $C_{3-7}$-Cycloalkylgruppe, die in 1-Stellung durch eine 5- bis 7-gliedrige Cycloalkyleniminocarbonylgruppe substituiert ist,

eine 4- bis 7-gliedrige Cycloalkyleniminocarbonylgruppe,

eine gegebenenfalls durch ein Fluor-, Chlor- oder Bromatom, durch eine Trifluormethyl-, $C_{1-3}$-Alkyl- oder $C_{1-3}$-Alkoxygruppe substituierte Phenylgruppe,

eine $C_{1-3}$-Alkoxy-, Phenyl-$C_{1-3}$-alkoxy-, Heteroaryloxy- oder Heteroaryloxy-$C_{1-3}$-alkoxygruppe, in der der Alkoxyteil jeweils in 2- oder 3-Stellung auch durch eine Amino-, $C_{1-3}$-Alkylaminooder Di-($C_{1-3}$-Alkyl)-aminogruppe substituiert sein kann,

eine $C_{3-7}$-Cycloalkoxygruppe, wobei die Methylengruppe in 3- oder 4-Stellung in einer $C_{5-7}$-Cycloalkoxygruppe durch eine -NH-Gruppe ersetzt sein kann, wobei die -NH-Gruppe durch eine Arylcarbonyl- oder Arylsulfonylgruppe, durch eine $C_{1-3}$-Alkylcarbonylgruppe, in welcher das Sauerstoffatom der Carbonylgruppe durch eine Iminogruppe ersetzt sein und der Alkanoylteil durch eine Amino-, $C_{1-3}$-Alkylamino- oder Di-($C_{1-3}$-Alkyl)-aminogruppe substituiert sein kann, oder durch eine $C_{1-3}$-Alkylgruppe, die in 2-oder 3-Stellung durch eine Amino-, $C_{1-3}$-Alkylamino- oder Di-($C_{1-3}$-Alkyl)-aminogruppe substituiert sein kann,

insbesondere bedeutet

$R_1$ eine durch eine 4- bis 7-gliedrige Cycloalkyleniminogruppe substituierte $C_{1-3}$-Alkylgruppe,

eine Amino-, $C_{1-5}$-Alkylamino-, $C_{5-7}$-Cycloalkylamino- oder Phenyl-$C_{1-3}$-alkylaminogruppe, die jeweils am Aminstickstoffatom durch eine Benzoyl- oder Phenylsulfonylgruppe oder durch eine gegebenenfalls im $C_{1-3}$-Alkylteil durch eine Carboxygruppe substituierte $C_{1-3}$-Alkyl- oder $C_{1-3}$-Alkylcarbonylgruppe substituiert sein kann,

eine durch eine $C_{1-3}$-Alkylgruppe substituierte 4- bis 7-gliedrige Cycloalkyleniminocarbonylgruppe,

eine gegebenenfalls durch eine $C_{1-3}$-Alkylgruppe substituierte 4-bis 7-gliedrige Cycloalkyleniminosulfonylgruppe,

eine gegebenenfalls durch eine oder zwei $C_{1-3}$-Alkylgruppen substituierte Aminosulfonylgruppe,

eine Aminosulfonylphenylgruppe,

eine durch ein Fluor-, Chlor- oder Bromatom, durch eine Trifluormethyl-, Aminosulfonyl-, $C_{1-3}$-Alkyl- oder $C_{1-3}$-Alkoxygruppe substituierte Phenylgruppe, die zusätzlich durch ein Fluor-, Chlor- oder Bromatom, durch eine Trifluormethyl-, $C_{1-3}$-Alkyl- oder $C_{1-3}$-Alkoxygruppe substituiert ist,

eine $C_{3-7}$-Cycloalkoxygruppe, wobei die Methylengruppe in 3- oder 4-Stellung in einer $C_{5-7}$-Cycloalkoxygruppe durch eine -NH-Gruppe ersetzt ist, wobei die -NH-Gruppe

durch eine Aminocarbonyl-, $C_{1-3}$-Alkylaminocarbonyl- oder Di-($C_{1-3}$-Alkyl)-aminocarbonylgruppe, in denen jeweils das Sauerstoffatom der Carbonylgruppe durch eine Iminogruppe ersetzt ist, substituiert ist.

Desweiteren schließen die bei der Definition der vorstehend erwähnten gesättigten Alkyl- und Alkoxyteile, die mehr als 2 Kohlenstoffatome enthalten, auch deren verzweigte Isomere wie beispielsweise die Isopropyl-, tert.Butyl-, Isobutylgruppe etc. ein.

Bevorzugte Verbindungen der obigen allgemeinen Formel I sind diejenigen, in denen

Ar eine gegebenenfalls durch ein Fluor-, Chlor- oder Bromatom, durch eine Methyl-, Hydroxy-, Methoxy- oder Benzyloxygruppe substitiuierte Phenylengruppe, welche durch eine weitere Methylgruppe substituiert sein kann,

$R_1$ eine gegebenenfalls durch ein Fluor-, Chlor- oder Bromatom, durch eine Trifluormethyl-, Aminosulfonyl-, $C_{1-3}$-Alkyl- oder $C_{1-3}$-Alkoxygruppe substituierte Phenylgruppe, die zusätzlich durch ein Fluor-, Chlor- oder Bromatom, durch eine Trifluormethyl-, $C_{1-3}$-Alkyl- oder $C_{1-3}$-Alkoxygruppe substituiert sein kann,

eine durch eine Dimethylamino-, Pyrrolidino- oder Imidazolylgruppe substituierte Methylgruppe, wobei der Imidazolylteil durch eine Methylgruppe substituiert sein kann,

eine Amino-, $C_{1-5}$-Alkylamino-, Cyclopentylamino- oder Benzylaminogruppe, die am Aminstickstoffatom durch eine Carboxy-$C_{1-2}$-alkyl-, $C_{1-3}$-Alkoxycarbonyl-$C_{1-2}$-alkyl-, Carboxy-$C_{1-2}$-alkylcarbonyl- oder $C_{1-3}$-Alkoxycarbonyl-$C_{1-2}$-alkylcarbonylgruppe substituiert sein kann,

eine Benzoylamino- oder Phenylsulfonylaminogruppe,

eine Cyclopropylgruppe, die in 1-Stellung durch eine 5- bis 7-gliedrige Cycloalkyleniminocarbonylgruppe substituiert ist,

eine gegebenenfalls durch eine Methylgruppe substituierte Pyrrolidinocarbonyl-, Piperidinocarbonyl-, Pyrrolidinosulfonyloder Piperidinosulfonylgruppe,

eine $C_{1-3}$-Alkoxygruppe, in der der Alkoxyteil jeweils in 2- oder 3-Stellung durch eine Amino-, $C_{1-3}$-Alkylamino- oder

Di-($C_{1-3}$-Alkyl)-aminogruppe substituiert sein kann,
eine Phenyl-$C_{1-3}$-alkoxy- oder Pyridinyloxygruppe,
eine $C_{5-7}$-Cycloalkoxygruppe, in der die Methylengruppe in 3- oder 4-Stellung durch eine -NH-Gruppe ersetzt sein kann, wobei die -NH-Gruppe

durch eine $C_{1-3}$-Alkyl- oder $C_{2-3}$-Alkanoylgruppe,

durch eine $C_{2-3}$-Alkanoyl- oder Aminocarbonylgruppe, in der jeweils das Sauerstoffatom der Carbonylgruppe durch eine Iminogruppe ersetzt ist, substituiert sein kann,

$R_2$ ein Wasserstoff-, Fluor-, Chlor- oder Bromatom, eine Methyl-, Hydroxy- oder Methoxygruppe,
$R_3$ ein Wasserstoffatom oder eine Methylgruppe,
$R_4$ ein Wasserstoffatom oder eine gegebenenfalls durch eine Carboxy- oder $C_{1-3}$-Alkoxycarbonylgruppe substituierte Methyl- oder Ethylgruppe und
$R_5$ eine Cyanogruppe oder eine gegebenenfalls durch eine $C_{1-6}$-Alkoxycarbonyl- oder Benzoylgruppe substituierte Amidinogruppe bedeuten,
deren Isomere und deren Salze.

Besonders bevorzugte Verbindungen der allgemeinen Formel I, sind diejenigen, in denen
Ar eine gegebenenfalls durch eine Methyl-, Hydroxy-, Methoxyoder Benzyloxygruppe substitiuierte Phenylengruppe,
$R_1$ eine gegebenenfalls durch ein Fluor-, Chlor- oder Bromatom, durch eine Trifluormethyl-, Aminosulfonyl-, $C_{1-3}$-Alkyl- oder $C_{1-3}$-Alkoxygruppe substituierte Phenylgruppe, die zusätzlich durch ein Fluor-, Chlor- oder Bromatom, durch eine Trifluormethyl-, $C_{1-3}$-Alkyl- oder $C_{1-3}$-Alkoxygruppe substituiert sein kann,
eine Cyclopropylgruppe, die in 1-Stellung durch eine 5- bis 7-gliedrige Cycloalkyleniminocarbonylgruppe substituiert ist, oder eine 4- bis 7-gliedrige Cycloalkyleniminocarbonylgruppe,
eine gegebenenfalls durch eine Methylgruppe substituierte Pyrrolidinocarbonyl-, Piperidinocarbonyl- oder Pyrrolidinosulfonylgruppe,
$R_2$ ein Wasserstoff-, Fluor-, Chlor- oder Bromatom oder eine Methylgruppe,
$R_3$ ein Wasserstoffatom oder eine Methylgruppe,
$R_4$ ein Wasserstoffmatom oder eine durch eine Carboxy-, Methoxycarbonyl- oder Ethoxycarbonylgruppe substituierte Methyloder Ethylgruppe und
$R_5$ eine gegebenenfalls durch eine $C_{1-6}$-Alkoxycarbonyl- oder Benzoylgruppe substituierte Amidinogruppe bedeuten,
deren Isomere und deren Salze.

Beispielsweise seien folgende bevorzugte Verbindungen erwähnt:

(a) 2-(5-Carbamimidoyl-2-hydroxy-phenyl)-N-[3-methyl-4-(pyrrolidin-1-yl-carbonyl)-phenyl]-acetamid,

(b) 2-(2-Benzyloxy-5-carbamimidoyl-phenyl)-N-(2-ethoxycarbonyl-ethyl)-N-[3-methyl-4-(pyrrolidin-3-yl-carbonyl)-phenyl]-acetamid,

(c) 2-(2-Hydroxy-5-carbamimidoyl-phenyl)-N-(2-ethoxycarbonylethyl)-N-[3-methyl-4-(pyrrolidin-1-yl-carbonyl)-phenyl]-acetamid,

(d) 2-(2-Hydroxy-5-carbamimidoyl-phenyl)-N-(2-carboxy-ethyl)-N-[3-methyl-4-(pyrrolidin-1-yl-carbonyl)-phenyl]-acetamid,

(e) 2-(5-Carbamimidoyl-2-hydroxy-phenyl)-N-(3-methyl-4-(piperidin-1-yl-carbonyl)-phenyl]-acetamid und

(f) 2-(5-Carbamimidoyl-2-hydroxy-phenyl)-N-[3-methyl-4-(2-aminosulfonyl-phenyl)-phenyl]-acetamid,

in denen die Amidinogruppe zusätzlich durch eine $C_{1-6}$-Alkoxycarbonyl- oder Benzoylgruppe substituiert sein kann, und deren Salze.

Erfindungsgemäß erhält man die Verbindungen der allgemeinen Formel I nach an sich bekannten Verfahren, beispielsweise nach folgenden Verfahren:

a) Acylierung einer Verbindung der allgemeinen Formel

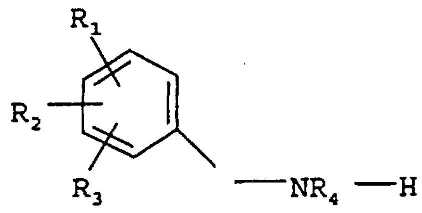

(II),

in der
$R_1$ bis $R_4$ wie eingangs erwähnt definiert sind, mit einer Carbonsäure der allgemeinen Formel

$$HO—CO—CH_2—Ar—R_5 \qquad (III),$$

in der
Ar, und $R_5$ wie eingangs erwähnt definiert sind, oder mit deren reaktionsfähigen Derivaten.

Die Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril oder Sulfolan gegebenenfalls in Gegenwart einer anorganischen oder organischen Base bei Temperaturen zwischen -20 und 200°C, vorzugsweise jedoch bei Temperaturen zwischen -10 und 160°C, durchgeführt.

Die Acylierung kann jedoch auch mit der freien Säure gegebenenfalls in Gegenwart eines die Säure aktivierenden Mittels oder eines wasserentziehenden Mittels, z.B. in Gegenwart von Chlorameisensäureisobutylester, Thionylchlorid, Trimethylchlorsilan, Chlorwasserstoff, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, bei Temperaturen zwischen -20 und 200°C, vorzugsweise jedoch bei Temperaturen zwischen -10 und 160°C, durchgeführt werden.

b) Zur Herstellung einer Verbindung der allgemeinen Formel I, in der $R_5$ eine Amidinogruppe, die durch eine oder zwei $C_{1-3}$-Alkylgruppen substituiert sein kann:

Umsetzung einer gegebenenfalls im Reaktionsgemisch gebildeten Verbindung der allgemeinen Formel

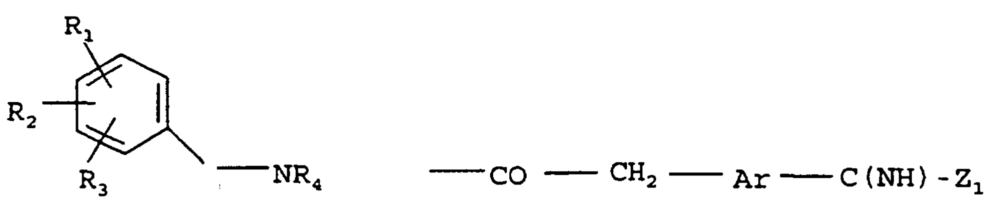

(IV),

in der
$R_1$ bis $R_4$, und Ar wie eingangs erwähnt definiert sind und $Z_1$ eine Alkoxy- oder Aralkoxygruppe wie die Methoxy-, Ethoxy-, n-Propoxy-, Isopropoxy- oder Benzyloxygruppe oder eine Alkylthio- oder Aralkylthiogruppe wie die Methylthio-, Ethylthio-, n-Propylthio- oder Benzylthiogruppe darstellt, mit einem Amin der allgemeinen Formel

$$H - R_6NR_7 , \qquad (V)$$

in der
$R_6$ und $R_7$, die gleich oder verschieden sein können, jeweils ein Wasserstoffatom oder eine $C_{1-3}$-Alkylgruppe bedeuten, oder mit dessen Salzen.

Die Umsetzung wird zweckmäßigerweise in einem Lösungsmittel wie Methanol, Ethanol, n-Propanol, Tetrahydrofuran oder Dioxan bei Temperaturen zwischen 0 und 150°C, vorzugsweise bei Temperaturen zwischen 0 und 80°C, mit einem Amin der allgemeinen Formel V oder mit einem entsprechenden Säureadditionssalz wie beispielsweise Ammoniumcarbonat oder Ammoniumacetat durchgeführt.

Eine Verbindung der allgemeinen Formel IV erhält man beispielsweise durch Umsetzung einer entsprechenden Cyanoverbindung mit einem entsprechenden Alkohol wie Methanol, Ethanol, n-Propanol, Isopropanol oder Benzylalkohol in Gegenwart einer Säure wie Salzsäure oder durch Umsetzung eines entsprechenden Amids mit einem Trialkyloxoniumsalz wie Triethyloxonium-tetrafluorborat in einem Lösungsmittel wie Methylenchlorid, Tetrahydrofuran oder Dioxan bei Temperaturen zwischen 0 und 50°C, vorzugsweise jedoch bei 20°C, oder eines entsprechenden Nitrils mit Schwefelwasserstoff zweckmäßigerweise in einem Lösungsmittel wie Pyridin oder Dimethylformamid und in Gegenwart einer Base wie Triethylamin und anschließender Alkylierung des gebildeten Thioamids mit einem entsprechenden Alkyl- oder Aralkylhalogenid.

Erhält man erfindungsgemäß eine Verbindung der allgemeinen Formel I, die eine Amino- oder Iminogruppe enthält, so kann diese anschließend mit einem entsprechenden Acylderivat in eine entsprechende Acylverbindung der allgemeinen Formel I übergeführt werden und/oder

eine Verbindung der allgemeinen Formel I, die eine veresterte Carboxygruppe enthält, so kann diese mittels Hydrolyse in eine entsprechende Carbonsäure der allgemeinen Formel I übergeführt werden und/oder

eine Verbindung der allgemeinen Formel I, die eine Carboxygruppe enthält, so kann diese anschließend mittels Veresterung in einen entsprechenden Ester übergeführt werden.

Die anschließende Acylierung wird zweckmäßigerweise mit einem entsprechenden Halogenid oder Anhydrid in einem Lösungsmittel wie Methylenchlorid, Chloroform, Tetrachlorkohlenstoff, Ether, Tetrahydrofuran, Dioxan, Benzol, Toluol, Acetonitril oder Sulfolan gegebenenfalls in Gegenwart einer anorganischen oder organischen Base bei Temperaturen zwischen -20 und 200°C, vorzugsweise jedoch bei Temperaturen zwischen -10 und 160°C, durchgeführt. Diese kann jedoch auch mit der freien Säure gegebenenfalls in Gegenwart eines die Säure aktivierenden Mittels oder eines wasserentziehenden Mittels, z.B. in Gegenwart von Chlorameisensäureisobutylester, Thionylchlorid, Trimethylchlorsilan, Chlorwasserstoff, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid oder 1-Hydroxy-benztriazol, N,N'-Carbonyldiimidazol oder N,N'-Thionyldiimidazol oder Triphenylphosphin/Tetrachlorkohlenstoff, bei Temperaturen zwischen -20 und 200°C, vorzugsweise jedoch bei Temperaturen zwischen -10 und 160°C, durchgeführt werden.

Die nachträgliche Hydrolyse wird zweckmäßigerweise entweder in Gegenwart einer Säure wie Salzsäure, Schwefelsäure, Phosphorsäure, Essigsäure, Trichloressigsäure, Trifluoressigsäure oder deren Gemischen oder in Gegenwart einer Base wie Lithiumhydroxid, Natriumhydroxid oder Kaliumhydroxid in einem geeigneten Lösungsmittel wie Wasser, Wasser/Methanol, Wasser/Ethanol, Wasser/Isopropanol, Methanol, Ethanol, Wasser/Tetrahydrofuran oder Wasser/Dioxan und die anschließende Decarboxylierung in Gegenwart einer Säure wie vorstehend beschrieben bei Temperaturen zwischen -10 und 120°C, z.B. bei Temperaturen zwischen Raumtemperatur und der Siedetemperatur des Reaktionsgemisches, durchgeführt.

Die nachträgliche Veresterung wird mit einem entsprechenden Alkohol zweckmäßigerweise in einem Lösungsmittel oder Lösungsmittelgemisch wie Methylenchlorid, Benzol, Toluol, Chlorbenzol, Tetrahydrofuran, Benzol/Tetrahydrofuran oder Dioxan, vorzugsweise jedoch in einem Überschuß des eingesetzten Alkohols, gegebenenfalls in Gegenwart einer Säure wie Salzsäure oder in Gegenwart eines wasserentziehenden Mittels, z.B. in Gegenwart von Chlorameisensäureisobutylester, Thionylchlorid, Trimethylchlorsilan, Salzsäure, Schwefelsäure, Methansulfonsäure, p-Toluolsulfonsäure, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol- oder N,N'-Thionyldiimidazol, Triphenylphosphin/Tetrachlorkohlenstoff oder Triphenylphosphin/Azodicarbonsäurediethylester gegebenenfalls in Gegenwart einer Base wie Kaliumcarbonat, N-Ethyl-diisopropylamin oder N,N-Dimethylamino-pyridin zweckmäßigerweise bei Temperaturen zwischen 0 und 150°C, vorzugsweise bei Temperaturen zwischen 0 und 80°C, oder mit einem entsprechenden Halogenid in einem Lösungsmittel wie Methylenchlorid, Tetrahydrofuran, Dioxan, Dimethylsulfoxid, Dimethylformamid oder Aceton gegebenenfalls in Gegenwart eines Reaktionsbeschleunigers wie Natrium- oder Kaliumiodid und vorzugsweise in Gegenwart einer Base wie Natriumcarbonat oder Kaliumcarbonat oder in Gegenwart einer tertiären organischen Base wie N-Ethyl-diisopropylamin oder N-Methyl-morphölin, welche gleichzeitig auch als Lösungsmittel dienen können, oder gegebenenfalls in Gegenwart von Silberkarbonat oder Silberoxid bei Temperaturen zwischen -30 und 100°C, vorzugsweise jedoch bei Temperaturen zwischen -10 und 80°C, durchgeführt.

Bei den vorstehend beschriebenen Umsetzungen können gegebenenfalls vorhandene reaktive Gruppen wie Hydroxy-, Carboxy-, Amino-, Alkylamino- oder Iminogruppen während der Umsetzung durch übliche Schutzgruppen geschützt werden, welche nach der Umsetzung wieder abgespalten werden.

Beispielsweise kommt als Schutzrest für eine Hydroxygruppe die Methoxy-, Benzyloxy-, Trimethylsilyl-, Acetyl-, Benzoyl-, tert.Butyl-, Trityl-, Benzyl- oder Tetrahydropyranylgruppe,

als Schutzreste für eine Carboxylgruppe die Trimethylsilyl-, Methyl-, Ethyl-, tert.Butyl-, Benzyl- oder Tetrahydropyranylgruppe und
als Schutzrest für eine Amino-, Alkylamino- oder Iminogruppe die Acetyl-, Trifluoracetyl-, Benzoyl-, Ethoxycarbonyl-, tert.Butoxycarbonyl-, Benzyloxycarbonyl-, Benzyl-, Methoxybenzyl- oder 2,4-Dimethoxybenzylgruppe und für die Aminogruppe zusätzlich die Phthalylgruppe in Betracht.

Die gegebenenfalls anschließende Abspaltung eines verwendeten Schutzrestes erfolgt beispielsweise hydrolytisch in einem wäßrigen Lösungsmittel, z.B. in Wasser, Isopropanol/Wasser, Tetrahydrofuran/Wasser oder Dioxan/Wasser, in Gegenwart einer Säure wie Trifluoressigsäure, Salzsäure oder Schwefelsäure oder in Gegenwart einer Alkalibase wie Lithiumhydroxid, Natriumhydroxid oder Kaliumhydroxid oder mittels Etherspaltung, z.B. in Gegenwart von Jodtrimethylsilan, bei Temperaturen zwischen 0 und 100°C, vorzugsweise bei Temperaturen zwischen 10 und 50°C.

Die Abspaltung eines Benzyl-, Methoxybenzyl- oder Benzyloxycarbonylrestes erfolgt jedoch beispielsweise hydrogenolytisch, z.B. mit Wasserstoff in Gegenwart eines Katalysators wie Palladium/Kohle in einem Lösungsmittel wie Methanol, Ethanol, Essigsäureethylester, Dimethylformamid, Dimethylformamid/Aceton oder Eisessig gegebenenfalls unter Zusatz einer Säure wie Salzsäure bei Temperaturen zwischen 0 und 50°C, vorzugsweise jedoch bei Raumtemperatur, und bei einem Wasserstoffdruck von 1 bis 7 bar, vorzugsweise jedoch von 3 bis 5 bar.

Die Abspaltung einer Methoxybenzylgruppe kann auch in Gegenwart eines Oxidationsmittels wie Cer(IV) ammoniumnitrat in einem Lösungsmittel wie Methylenchlorid, Acetonitril oder Acetonitril/Wasser bei Temperaturen zwischen 0 und 50°C, vorzugsweise jedoch bei Raumtemperatur, erfolgen.

Die Abspaltung einer Methoxy erfolgt zweckmäßigerweise in Gegenwart Bortribromid in einem Lösungsmittel wie Methylenchlorid bei Temperaturen zwischen -35 und -25°C.

Die Abspaltung eines 2,4-Dimethoxybenzylrestes erfolgt jedoch vorzugsweise in Trifluoressigsäure in Gegenwart von Anisol.

Die Abspaltung eines tert.Butyl- oder tert.Butyloxycarbonylrestes erfolgt vorzugsweise durch Behandlung mit einer Säure wie Trifluoressigsäure oder Salzsäure gegebenenfalls unter Verwendung eines Lösungsmittels wie Methylenchlorid, Dioxan oder Ether.

Die Abspaltung eines Phthalylrestes erfolgt vorzugsweise in Gegenwart von Hydrazin oder eines primären Amins wie Methylamin, Ethylamin oder n-Butylamin in einem Lösungsmittel wie Methanol, Ethanol, Isopropanol, Toluol/Wasser oder Dioxan bei Temperaturen zwischen 20 und 50°C.

Die Abspaltung eines Allyloxycarbonylrestes erfolgt durch Behandlung mit einer katalytischen Menge Tetrakis-(triphenylphosphin)-palladium(O) vorzugsweise in einem Lösungsmittel wie Tetrahydrofuran und vorzugsweise in Gegenwart eines Überschusses von einer Base wie Morpholin oder 1,3-Dimedon bei Temperaturen zwischen 0 und 100°C, vorzugsweise bei Raumtemperatur und unter Inertgas, oder durch Behandlung mit einer katalytischen Menge von Tris-(triphenylphosphin)-rhodium(I)-chlorid in einem Lösungsmittel wie wässrigem Ethanol und gegebenenfalls in Gegenwart einer Base wie 1,4-Diazabicyclo[2.2.2]octan bei Temperaturen zwischen 20 und 70°C.

Die als Ausgangsstoffe verwendeten Verbindungen der allgemeinen Formeln II bis V, welche teilweise literaturbekannt sind, erhält man nach literaturbekannten Verfahren, desweiteren wird ihre Herstellung in den Beispielen beschrieben.

Die Chemie der Verbindungen der allgemeinen Formel II wird beispielsweise von Schröter in Stickstoffverbindungen II, Seiten 341-730, Methoden der organischen Chemie (Houben-Weyl), 4. Auflage, Verlag Thieme, Stuttgart 1957, und die der allgemeinen Formel III von J.F. Hartwig in Angew. Chem. 110, 2154-2157 (1998) beschrieben.

Ferner können die erhaltenen Verbindungen der allgemeinen Formel I in ihre Enantiomeren und/oder Diastereomeren aufgetrennt werden.

So lassen sich beispielsweise die erhaltenen Verbindungen der allgemeinen Formel I, welche in Racematen auftreten, nach an sich bekannten Methoden (siehe Allinger N. L. und Eliel E. L. in "Topics in Stereochemistry", Vol. 6, Wiley Interscience, 1971) in ihre optischen Antipoden und Verbindungen der allgemeinen Formel I mit mindestes 2 asymmetrischen Kohlenstoffatomen auf Grund ihrer physikalisch-chemischen Unterschiede nach an sich bekannten Methoden, z.B. durch Chromatographie und/oder fraktionierte Kristallisation, in ihre Diastereomeren auftrennen, die, falls sie in racemischer Form anfallen, anschließend wie oben erwähnt in die Enantiomeren getrennt werden können.

Die Enantiomerentrennung erfolgt vorzugsweise durch Säulentrennung an chiralen Phasen oder durch Umkristallisieren aus einem optisch aktiven Lösungsmittel oder durch Umsetzen mit einer, mit der racemischen Verbindung Salze oder Derivate wie z.B. Ester oder Amide bildenden optisch aktiven Substanz, insbesondere Säuren und ihre aktivierten Derivate oder Alkohole, und Trennen des auf diese Weise erhaltenen diastereomeren Salzgemisches oder Derivates, z.B. auf Grund von verschiedenen Löslichkeiten, wobei aus den reinen diastereomeren Salzen oder Derivaten die freien Antipoden durch Einwirkung geeigneter Mittel freigesetzt werden können. Besonders gebräuchliche, optisch aktive Säuren sind z.B. die D- und L-Formen von Weinsäure oder Dibenzoylweinsäure, Di-o-Tolylweinsäure, Apfelsäure, Mandelsäure, Camphersulfonsäure, Glutaminsäure, Asparaginsäure oder Chinasäure. Als optisch aktiver Alkohol kommt beispielsweise (+)- oder (-)-Menthol und als optisch aktiver Acylrest in Amiden beispielsweise der (+)- oder (-)-Menthyloxycarbonylrest in Betracht.

Desweiteren können die erhaltenen Verbindungen der Formel I in ihre Salze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Methansulfonsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der Formel I, falls diese eine Carboxygruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Ethanolamin, Diethanolamin und Triethanolamin in Betracht.

Wie bereits eingangs erwähnt, weisen die neuen Verbindungen der allgemeinen Formel I und deren Salze wertvolle Eigenschaften auf. So stellen die Verbindungen der allgemeinen Formel I, in denen $R_5$ eine Cyanogruppe darstellt, wertvolle Zwischenprodukte zur Herstellung der übrigen Verbindungen der allgemeinen Formel I dar, und die Verbindungen der allgemeinen Formel I, in denen $R_5$ eine der eingangs erwähnten Amidinogruppen darstellt, sowie deren Tautomeren, deren Stereoisomeren und deren physiologisch verträglichen Salze weisen wertvolle pharmakologische Eigenschaften auf, insbesondere eine antithrombotische Wirkung, welche vorzugsweise auf einer Thrombin oder Faktor Xa beeinflussenden Wirkung beruht, beispielsweise auf einer thrombinhemmenden oder Faktor Xa-hemmenden Wirkung, auf einer die aPTT-Zeit verlängernden Wirkung und auf einer Hemmwirkung auf verwandte Serinproteasen wie z. B. Trypsin, Urokinase Faktor VIIa, Faktor IX, Faktor XI und Faktor XII.

Beispielsweise wurden die Verbindungen

A = 2-(5-Carbamimidoyl-2-hydroxy-phenyl)-N-[3-methyl-4-(pyrrolidin-1-yl-carbonyl)-phenyl]-acetamid-hydrochlorid,

B = 2-(2-Benzyloxy-5-carbamimidoyl-phenyl)-N-(2-ethoxycarbonyl-ethyl)-N-[3-methyl-4-(pyrrolidin-1-yl-carbonyl)-phenyl]-acetamid-hydrochlorid,

C = 2-(2-Hydroxy-5-carbamimidoyl-phenyl)-N-(2-ethoxycarbonylethyl)-N-[3-methyl-4-(pyrrolidin-1-yl-carbonyl)-phenyl]-acetamid-hydrochlorid,

D = 2-(2-Hydroxy-5-carbamimidoyl-phenyl)-N-(2-carboxy-ethyl)-N-[3-methyl-4-(pyrrolidin-1-yl-carbonyl)-phenyl]-acetamidhydrochlorid

E = 2-(5-Carbamimidoyl-2-hydroxy-phenyl)-N-[3-methyl-4-(piperidin-1-yl-carbonyl)-phenyl]-acetamid-hydrochlorid und

F = 2-(5-Carbamimidoyl-2-hydroxy-phenyl)-N-[3-methyl-4-(2-aminosulfonyl-phenyl)-phenyl]-acetamid-hydrochlorid,

auf ihre Wirkung auf die Hemmung des Faktors Xa wie folgt untersucht:

Methodik: Enzymkinetische Messung mit chromogenem Substrat. Die durch humanen Faktor Xa aus dem farblosen chromogenen Substrat freigesetzte Menge anp-Nitroanilin (pNA) wird photometrisch bei 405 nm bestimmt. Sie ist proportional der Aktivität des eingesetzten Enzyms. Die Hemmung der Enzymaktivität durch die Testsubstanz (bezogen auf die Lösungsmittelkontrolle) wird bei verschiedenen Testsubstanz-Konzentrationen ermittelt und hieraus die $IC_{50}$ berechnet als diejenige Konzentration, die den eingesetzten Faktor Xa um 50 % hemmt.

Material:
Tris(hydroxymethyl)-aminomethan-Puffer (100 mMol) und Natriumchlorid (150 mMol), pH 8.0
Faktor Xa (Roche), Spez. Aktivität: 10 U/0.5 ml, Endkonzentration: 0.175 U/ml pro Reaktionsansatz
Substrat Chromozym X (Roche), Endkonzentration: 200 µMol/l pro Reaktionsansatz
Testsubstanz: Endkonzentration 100, 30, 10, 3, 1, 0.3, 0.1, 0.03, 0.01, 0.003, 0.001 µMol/l

Durchführung: 10 µl einer 23.5-fach konzentrierteren Ausgangslösung der Testsubstanz bzw. Lösungsmittel (Kontrolle), 175 µl Tris(hydroxymethyl)-aminomethan-Puffer und 25 µl Faktor Xa-Gebrauchslösung von 1.65 U/ml werden 10 Minuten bei 37°C inkubiert. Nach Zugabe von 25 µl Chromozym X-Gebrauchslösung (1.88 µMol/l) wird die Probe im Photometer (SpectraMax 250) bei 405 nm für 150 Sekunden bei 37°C gemessen.

Auswertung:

1. Ermittlung der maximalen Zunahme (deltaOD/Minuten) über 3 Messpunkte.

2. Ermittlung der %-Hemmung bezogen auf die Lösungsmittelkontrolle.

3. Erstellen einer Dosiswirkungskurve (%-Hemmung vs Substanzkonzentration).

4. Ermittlung der $IC_{50}$ durch Interpolation des X-Wertes (Substanzkonzentration) der Dosiswirkungskurve bei Y = 50 % Hemmung.

Die nachfolgende Tabelle enthält die gefundenen Werte:

| Substanz | Hemmung des Faktors Xa ($IC_{50}$ in µM) |
|---|---|
| A | 0.030 |
| B | 0.680 |
| C | 0.120 |
| D | 0.850 |
| E | 0.085 |
| F | 0.260 |

Die erfindungsgemäß hergestellten Verbindungen sind gut verträglich, da bei therapeutischen Dosen keine toxischen Nebenwirkungen beobachtet werden konnten.

Aufgrund ihrer pharmakologischen Eigenschaften eignen sich die neuen Verbindungen und deren physiologisch verträglichen Salze zur Vorbeugung und Behandlung venöser und arterieller thrombotischer Erkrankungen, wie zum Beispiel der Behandlung von tiefen Beinvenen-Thrombosen, der Verhinderung von Reocclusionen nach Bypass-Operationen oder Angioplastie (PT(C)A), sowie der Occlusion bei peripheren arteriellen Erkrankungen wie Lungenembolie, der disseminierten intravaskulären Gerinnung, der Prophylaxe der Koronarthrombose, der Prophylaxe des Schlaganfalls und der Verhinderung der Occlusion von Shunts. Zusätzlich sind die erfindungsgemäßen Verbindungen zur antithrombotischen Unterstützung bei einer thrombolytischen Behandlung, wie zum Beispiel mit rt-PA oder Streptokinase, zur Verhinderung der Langzeitrestenose nach PT(C)A, zur Verhinderung der Metastasierung und des Wachstums von koagulationsabhängigen Tumoren und von fibrinabhängigen Entzündungsprozessen, z.B. bei der Behandlung der pulmonaren Fibrosis, geeignet.

Die zur Erzielung einer entsprechenden Wirkung erforderliche Dosierung beträgt zweckmäßigerweise bei intravenöser Gabe 0,1 bis 30 mg/kg, vorzugsweise 0,3 bis 10 mg/kg, und bei oraler Gabe 0,1 bis 50 mg/kg, vorzugsweise 0,3 bis 30 mg/kg, jeweils 1 bis 4 x täglich. Hierzu lassen sich die erfindungsgemäß hergestellten Verbindungen der Formel I, gegebenenfalls in Kombination mit anderen Wirksubstanzen, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Ethanol, Wasser/Glycerin, Wasser/Sorbit, Wasser/Polyethylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Die nachfolgenden Beispiele sollen die Erfindung näher erläutern:

Beispiel 1

2-(3-Carbamimidoyl-phenyl)-N-[2-chlor-5-(1-(pyrrolidin-1-ylcarbonyl)-cyclopropyl)-phenyl]-acetamid-hydrochlorid

a. 1-(4-Chlor-3-nitro-phenyl)-cyclopropancarbonsäure

350 ml rauchende Salpetersäure werden bei -25 bis -30°C portionsweise mit 50.0 g (0.21 Mol) 1-(4-Chlorphenyl)-cyclopropancarbonsäure versetzt. Nach beendeter Zugabe wird noch 15 Minuten bei -25°C gerührt und anschließend auf Eis gegossen. Die ausgefallene Substanz wird abgesaugt, mit Wasser gewaschen und getrocknet.

Ausbeute: 58.5 g (95 % der Theorie),
$R_f$-Wert: 0.43 (Kieselgel; Methylenchlorid/Methanol = 9.5:0.5)

b. 5-[1-(Pyrrolidin-1-yl-carbonyl)-cyclopropyl]-2-chlor-nitrobenzol

2.4 g (0.01 Mol) 1-(4-Chlor-3-nitro-phenyl)-cyclopropancarbonsäure werden in 25 ml Tetrahydrofuran gelöst und nach Zugabe von 3.2 g (0.01 Mol) O-(Benzotriazol-1-yl)-N,N,N',N'-tetramethyluroniumtetrafluorborat, 1.1 ml (0.01 Mol) N-Methyl-morpholin und 1.0 ml (0.012 Mol) Pyrrolidin 16 Stunden bei Raumtemperatur gerührt. Das Solvens wird abdestilliert, der Rückstand auf Eiswasser gegossen, mit Ammoniak alkalisch gestellt und mit Essigester extrahiert. Die organische Phase wird getrocknet und eingedampft.
Ausbeute: 2.5 g (85 % der Theorie),
$R_f$-Wert: 0.18 (Kieselgel; Cyclohexan/Essigester = 1:1)

c. 5-[1-(Pyrrolidin-1-yl-carbonyl)-cyclopropyl]-2-chlor-anilin

1.8 g (8.14 mMol) 5-[1-(Pyrrolidin-1-yl-carbonyl)-cyclopropyl]-2-chlor-nitrobenzol werden in 30 ml Essigester und 30 ml Ethanol gelöst und nach Zugabe von 0.8 g Palladium auf Aktivkohle (10%ig) 3 Stunden bei Raumtemperatur mit Wasserstoff hydriert. Anschließend wird vom Katalysator abfiltriert und eingedampft.
Ausbeute: 2.0 g (92.8 % der Theorie),
$R_f$-Wert: 0.24 (Kieselgel; Cyclohexan/Essigester/Ammoniak = 1:1:0.01)
$C_{14}H_{17}ClN_2O$ (264.75)
Massenspektrum: $M^+$ = 264/6 (Cl)

d. 2-(3-Cyano-phenyl)-N-[2-chlor-5-(1-(pyrrolidin-1-yl-carbonyl)-cyclopropyl)-phenyl]-acetamid

Hergestellt analog Beispiel 1b aus 5-[1-(Pyrrolidin-1-yl-carbonyl)-cyclopropyl]-2-chlor-anilin, O-(Benzotriazol-1-yl)-N,N,N',N'-tetramethyluroniumtetrafluorborat, N-Methyl-morpholin und 3-Cyanophenylessigsäure in Dimethylformamid.
Ausbeute: 43 % der Theorie,
$R_f$-Wert: 0.21 (Kieselgel; Cyclohexan/Essigester = 1:2)

e. 2-(3-Carbamimidoyl-phenyl)-N-[2-chlor-5-(1-(pyrrolidin-1-yl-carbonyl)-cyclopropyl)-phenyl]-acetamid-hydrochlorid

400 mg (0.1 mMol) 2-(3-Cyano-phenyl)-N-[2-chlor-5-(1-(pyrrolidin-1-yl-carbonyl)-cyclopropyl)-phenyl]-acetamid werden in 60 ml gesättigter ethanolischer Salzsäure gelöst und 17 Stunden bei Raumtemperatur gerührt. Das Solvens wird abdestilliert, der Rückstand in 50 ml absolutem Ethanol gelöst und mit 1.5 g (15.6 mMol) Ammoniumcarbonat versetzt. Nach 22 Stunden bei Raumtemperatur wird zur Trockene eingedampft. Der Rückstand wird an Kieselgel chromatographiert, wobei mit Methylenchlorid/Methanol/Eisessig (9:1:0.01) eluiert wird.
Ausbeute: 50 mg (11 % der Theorie),
$R_f$-Wert: 0.59 (Kieselgel; Methylenchlorid/Methanol/Ammoniak = 4:1:0.01)
$C_{23}H_{25}ClN_4O_2$ x HCl (424.94/461.4)
Massenspektrum: $(M+H)^+$ = 425/7 (Cl)

Referenz beispiel 2

3-Carbamimidoyl-N-[3-(1-(pyrrolidin-1-yl-carbonyl)-cyclopropyl)-benzyl]-benzamid-hydrochlorid

a. 1-(3-Brom-phenyl)-1-cyclopropan-nitril

25 g (0.13 Mol) 3-Brom-benzylcyanid werden in 32 ml (0.38 Mol) 1-Brom-2-chlor-ethan aufgenommen und mit 0.6 g (2.6 mMol) Benzyltriethylammoniumchlorid versetzt. Anschließend wird eine Lösung von 105.8 g (2.65 Mol) Natriumhydroxid in 106 ml Wasser bei 10 bis 25°C zugetropft. Nach 20 Stunden bei 55°C wird die Reaktionslösung auf Eiswasser gegossen und mit Essigester extrahiert. Die organischen Extrakte werden getrocknet und eingedampft. Der Rückstand wird mit Petrolether verrieben, abgesaugt und getrocknet.
Ausbeute: 19.3 g (68 % der Theorie),
$R_f$-Wert: 0.69 (Petrolether/Essigester = 4:1)

b. 1-(3-Brom-phenyl)-cyclopropancarbonsäure

7.6 g (0.135 Mol) Kaliumhydroxid werden in 60 ml Ethylenglykol gelöst, portionsweise mit 10.0 g (0.045 Mol) 1-(3-Brom-phenyl)-1-cyclopropan-nitril versetzt und nach Zugabe von 30 ml Wasser 4.5 Stunden auf 140°C erhitzt. Nach Abkühlung wird auf 600 ml Eiswasser gegossen und mit Ether extrahiert. Die wässrige Phase wird auf Eis/konz. Salzsäure gegossen, das ausgefallene Produkt abgesaugt und getrocknet.
Ausbeute: 10.1 g (93 % der Theorie),
$R_f$-Wert: 0.85 (Kieselgel; Cyclohexan/Essigester/Eisessig = 1:1:0.01)

c. 3-[1-(Pyrrolidin-1-yl-carbonyl)-cyclopropyl]-brom-benzol

Hergestellt analog Beispiel 1b aus 1-(3-Brom-phenyl)-cyclopropancarbonsäure, Pyrrolidin, O-(Benzotriazol-1-yl)-N,N,N',N'tetramethyluroniumtetrafluorborat und N-Methyl-morpholin in Tetrahydrofuran.
Ausbeute: 98 % der Theorie,
$R_f$-Wert: 0.55 (Kieselgel; Cyclohexan/Essigester/Eisessig = 1:1:0.01)

d. 3-[1-(Pyrrolidin-1-yl-carbonyl)-cyclopropyl]-benzonitril

6 g (20.4 mMol) 3-[1-(Pyrrolidin-1-yl-carbonyl)-cyclopropyl]-brom-benzol werden in 25 ml Dimethylformamid gelöst und nach Zugabe von 2.7 g (30.6 mMol) Kupfer-I-cyanid, 0.3 g (0.216 mMol) Tetrakis-triphenylphosphin-palladium-(0) und 5 g Aluminiumoxid 30 Stunden bei 140°C gerührt. Das unlösliche Material wird abfiltriert und die Lösung eingedampft. Der Rückstand wird an Kieselgel chromatographiert, wobei mit Cyclohexan/Essigester (1:2) eluiert wird.
Ausbeute: 1.8 g (36 % der Theorie),
$R_f$-Wert: 0.32 (Kieselgel; Cyclohexan/Essigester/Eisessig = 1:1:0.01)

e. 3-[1-(Pyrrolidin-1-yl-carbonyl)-cyclopropyl]-benzylamin

1.8 g (7.5 mMol) 3-[1-(Pyrrolidin-1-yl-carbonyl)-cyclopropyl]-benzonitril werden in 50 ml methanolischem Ammoniak unter Zugabe von 300 mg Raney-Nickel 3 Stunden bei 70°C mit Wasserstoff hydriert. Anschließend wird vom Katalysator abfiltriert und eingedampft.
Ausbeute: 1.8 g (98 % der Theorie),
$R_f$-Wert: 0.94 (Kieselgel; Methylenchlorid /Methanol/Ammoniak = 4:1:0.01)

f. 3-Cyano-N-[3-(1-(pyrrolidin-1-yl-carbonyl)-cyclopropyl)-benzyl]-benzamid

Hergestellt analog Beispiel 1b aus 3-[1-(Pyrrolidin-1-ylcarbonyl)-cyclopropyl]-benzylamin, 3-Cyanobenzoesäure, O-(Benzotriazol-1-yl)-N,N,N',N'-tetramethyluroniumtetrafluorborat und N-Methyl-morpholin in Tetrahydrofuran.
Ausbeute: 96 % der Theorie,
$R_f$-Wert: 0.56 (Kieselgel; Essigester/Ethanol = 9:1)

g. 3-Carbamimidoyl-N-[3-(1-(pyrrolidin-1-yl-carbonyl)-cyclopropyl)-benzyl]-benzamid-hydrochlorid

Hergestellt analog Beispiel 1e aus 3-Cyano-N-[3-(1-(pyrrolidin-1-yl-carbonyl)-cyclopropyl)-benzyl]-benzamid und Salzsäure/Ammoniumcarbonat in Ethanol.
Ausbeute: 58 % der Theorie,
$R_f$-Wert: 0.19 (Reversed Phase RP 8; 5%ige Natriumchlorid-Lösung/Methanol = 1:1)
$C_{23}H_{26}N_4O_2$ x HCl (390.48/426.95)
Massenspektrum: $(M+H)^+$ = 391
$(M-H+HCl)^-$ = 425/7 (Cl)

Beispiel 3

2-(5-Carbamimidoyl-2-hydroxy-phenyl)-N-[2-methyl-5-(1-(pyrrolidin-1-carbonyl)-cyclopropyl)-phenyl]-acetamid-hydrochlorid

a. 5-Cyano-2-methoxy-phenylessigsäure

Hergestellt analog Beispiel 2d aus 5-Brom-2-methoxy-phenylessigsäure, Kupfer-I-cyanid, Tetrakis-triphenylphosphin-palladium-(0) und Aluminiumoxid in Dimethylformamid.
Ausbeute: 37 % der Theorie,
$R_f$-Wert: 0.26 (Kieselgel; Cyclohexan/Essigester/Eisessig = 1:1:0.01)

b. 2-(5-Cyano-2-methoxy-phenyl)-N-[2-methyl-5-(1-(pyrrolidin-1-carbonyl)-cyclopropyl)-phenyl]-acetamid

0.6 g (3.3 mMol) 5-Cyano-2-methoxy-phenylessigsäure werden in 10 ml Dimethylformamid gelöst und nach Zugabe von 0.5 g (3.3 mMol) N,N-Carbonyldiimidazol 10 Minuten bei Raumtemperatur gerührt. Anschließend werden 0.8 g (3.3 mMol) 5-(Pyrrolidin-1-yl-carbonyl)-cyclopropyl)-2-methyl-anilin zugegeben. Das Reaktionsgemisch wird 4 Stunden bei 80°C gerührt, auf Raumtemperatur abgekühlt, mit Eiswasser versetzt, mit Ammoniak alkalisch gestellt und mehrfach mit Essigester extrahiert. Die vereinigten organischen Extrakte werden getrocknet und eingedampft. Der Rückstand wird an Kieselgel chromatographiert, wobei mit Cyclohexan/Essigester (7:3) eluiert wird.
Ausbeute: 73 % der Theorie,
$R_f$-Wert: 0.30 (Kieselgel; Essigester)

c. 2-(5-Cyano-2-hydroxy-phenyl)-N-[2-methyl-5-(1-(pyrrolidin-1-carbonyl)-cyclopropyl)-phenyl]-acetamid

0.7 g (1.67 mMol) 2-(5-Cyano-2-methoxy-phenyl)-N-[2-methyl-5-(1-(pyrrolidin-1-carbonyl)-cyclopropyl)-phenyl]-acetamid werden in 35 ml Methylenchlorid gelöst und bei -35 bis -25°C tropfenweise mit 10 ml einer 1-molaren Lösung von Bortribromid in Methylenchlorid (10 mMol) versetzt. Nach 1-stündigem Rühren bei 20°C wird zunächst mit Eis versetzt, anschließend werden 20 ml 2N Salzsäure zugegeben. Die wäßrige Phase wird mehrfach mit Methylenchlorid extrahiert, die vereinigten organischen Extrakte werden getrocknet und eingedampft. Der Rückstand wird an Kieselgel chromatographiert, wobei mit Methylenchlorid/-Ethanol (100:1) eluiert wird.
Ausbeute: 81 % der Theorie,
$R_f$-Wert: 0.14 (Kieselgel; Methylenchlorid/Ethanol = 49:1)

d. 2-(5-Carbamimidoyl-2-hydroxy-phenyl)-N-[2-methyl-5-(1-(pyrrolidin-1-carbonyl)-cyclopropyl)-phenyl]-acetamid-hydrochlorid

Hergestellt analog Beispiel 1e aus 2-(5-Cyano-2-hydroxy-phenyl)-N-[2-methyl-5-(1-(pyrrolidin-1-carbonyl)-cyclopropyl)-phenyl]-acetamid, und Salzsäure/Ammoniumcarbonat in Ethanol. Ausbeute: 80 % der Theorie,
$R_f$-Wert: 0.39 (Kieselgel; Methylenchlorid/Methanol/Eisessig = 4:1:0.01)
$C_{24}H_{28}N_4O_3$ x HCl (420.51/456.98)
Massenspektrum: $(M+H)^+$ = 421
$(M+Cl)^-$ = 455/7 (Cl)

Analog Beispiel 3 wird folgende Verbindung hergestellt:

(1) 2-(5-Carbamimidoyl-2-methoxy-phenyl)-N-[2-methyl-5-(1-(pyrrolidin-1-carbonyl)-cyclopropyl)-phenyl]-acetamidhydrochlorid
Ausbeute: 92 % der Theorie,
$R_f$-Wert: 0.33 (Kieselgel; Methylenchlorid/Methanol/Eisessig = 4:1:0.01)
$C_{25}H_{30}N_4O_3$ x HCl (434.55/471.01)
Massenspektrum: $(M+H)^+$ = 435

Beispiel 4

2-(5-Carbamimidoyl-2-hydroxy-phenyl)-N-[3-methyl-4-(pyrrolidin-1-yl-carbonyl)-phenyl]-acetamid-hydrochlorid

a. 2-(5-Cyano-2-methoxy-phenyl)-N-[3-methyl-4-(pyrrolidin-1-yl-carbonyl)-phenyl]-acetamid

0.4 g (2 mMol) 3-Methyl-4-(pyrrolidin-1-yl-carbonyl)-anilin werden in 15 ml Tetrahydrofuran gelöst und nach Zugabe von 0.3 ml (2 mMol) Triethylamin und 0.4 g (2 mMol) 5-Cyano-3-methoxy-phenylessigsäurechlorid 48 Stunden bei Raumtemperatur gerührt. Danach wird mit Wasser versetzt, mit Ammoniak alkalisch gestellt und mit Essigester extrahiert. Die vereinigten organischen Extrakte werden mit 1N Salzsäure gewaschen, getrocknet und eingedampft.
Ausbeute: 0.45 g (59 % der Theorie),
$R_f$-Wert: 0.18 (Kieselgel; Essigester)

b. 2-(5-Carbamimidoyl-2-methoxy-phenyl)-N-[3-methyl-4-(pyrrolidin-1-yl-carbonyl)-phenyl]-acetamid-hydrochlorid

Hergestellt analog Beispiel 1e aus 2-(5-Cyano-2-methoxyphenyl)-N-[3-methyl-4-(pyrrolidin-1-yl-carbonyl)-phenyl]-acetamid und Salzsäure/Ammoniumcarbonat in Ethanol.
Ausbeute: 36 % der Theorie,
$R_f$-Wert: 0.33 (Reversed Phase RP 8; 5%ige Natriumchlorid-Lösung/Methanol = 1:1)
$C_{22}H_{26}N_4O_3$ x HCl (394.48/430.94)
Massenspektrum: $(M+H)^+$ = 395
$(M-H+HCl)^-$ = 429

c. 2-(5-Carbamimidoyl-2-hydroxy-phenyl)-N-[3-methyl-4-(pyrrolidin-1-yl-carbonyl)-phenyl]-acetamid-hydrochlorid

Hergestellt analog Beispiel 3c aus 2-(5-Carbamimidoyl-2-methoxy-phenyl)-N-[3-methyl-4-(pyrrolidin-1-yl-carbonyl)-phenyl]-acetamid-hydrochlorid und Bortribromid in Dichlormethan.
Ausbeute: 19 % der Theorie,
$R_f$-Wert: 0.38 (Reversed Phase RP 8; 5%ige Natriumchlorid-Lösung/Methanol = 1:1)
$C_{21}H_{24}N_4O_3$ x HCl (380.45/416.91)
Massenspektrum: $(M+H)^+$ = 381
$(M-H)^-$ = 379

Analog Beispiel 4 werden folgende Verbindungen hergestellt:

(1) 2-(3-Carbamimidoyl-phenyl)-N-[3-methyl-4-(pyrrolidin-1-ylcarbonyl)-phenyl]-acetamid-hydrochlorid
Ausbeute: 12 % der Theorie,
$C_{21}H_{24}N_4O_2$ x HCl (364.45/400.92)
Massenspektrum: $(M+H)^+$ = 365

(2) 2-(5-Carbamimidoyl-2-hydroxy-phenyl)-N-methyl-N-[3-methyl-4-(pyrrolidin-1-yl-carbonyl)-phenyl]-acetamid-hydrochlorid Ausbeute: 99 % der Theorie,
$C_{22}H_{26}N_4O_3$ x HCl (394.48/430.94)
Massenspektrum: $(M+H)^+$ = 395
$(M-H)^-$ = 393

(3) 2- (5-Carbamimidoyl-2-benzyloxy-phenyl)-N-methyl-N-[3-methyl-4-(pyrrolidin-1-yl-carbonyl)-phenyl]-acetamid-hydrochlorid
Ausbeute: 90 % der Theorie,
$C_{29}H_{32}N_4O_3$ x HCl (484.60/521.06)
Massenspektrum: $(M+H)^+$ = 485
$(M-H+HCl)^-$ = 519/21 (Cl)

(4) 2-(5-Carbamimidoyl-2-hydroxy-phenyl)-N-[3-methyl-4-(N-(3-ethoxycarbonyl-propionyl)-N-cyclopentyl-amino)-phenyl]-acetamid-hydrochlorid
Ausbeute: 74% der Theorie,
$C_{27}H_{34}N_4O_5$ x HCl (494.61/531.06)
$R_f$-Wert: 0.36 (Reversed Phase RP8; 5%ige Kochsalzlösung/Methanol = 4:6)
Massenspektrum: $(M+H)^+$ = 495
$(M+Cl)^-$ = 529/531 (Cl
$(M-H)^-$ = 493

(5) 2-(5-Carbamimidoyl-2-benzyloxy-phenyl)-N-[3-methyl-4-(N-(3-ethoxycarbonyl-propionyl-N-(2-methyl-propyl)-amino)-phenyl]-acetamid-hydrochlorid
Ausbeute: 74% der Theorie,
$R_f$-Wert: 0.21 (Kieselgel; Methylenchlorid/Ethanol = 4:1) $C_{33}H_{40}N_4O_5$ x HCl (572.71/609.18)
Massenspektrum: $(M+H)^+$ = 573
$(M-H)^-$ = 571

(6) 2-(5-Carbamimidoyl-2-hydroxy-phenyl)-N-[3-methyl-4-(N-(3-ethoxycarbonyl-propionyl)-N-(2-methyl-propyl)-amino)-phenyl]-acetamid-hydrochlorid
Ausbeute: 100% der Theorie,

$R_f$-Wert: 0.33 (Reversed Phase RP8; 5%ige Kochsalzlösung/Methanol = 4:6)
$C_{26}H_{34}N_4O_5$ x HCl (482.58/519.05)
Massenspektrum: $(M+H)^+$ = 483
$(M-H)^-$ = 481
$(M+Cl)^-$ = 517/519 (Cl)

(7) 2-(5-Carbamimidoyl-2-hydroxy-phenyl)-N-[3-methyl-4-(N-ethoxycarbonylacetyl-N-cyclopentyl-amino)-phenyl]-acetamid-hydrochlorid

(8) 2-(5-Carbamimidoyl-2-hydroxy-phenyl)-N-[3-methyl-4-(pyrrolidin-1-yl-sulfonyl)-phenyl]-acetamid-hydrochlorid

Beispiel 5

2-(5-Carbamimidoyl-2-hydroxy-phenyl)-N-[2,5-dimethyl-4-(pyrrolidin-1-yl-carbonyl)-phenyl]-acetamid-hydrochlorid

a. 2,5-Dimethyl-4-(pyrrolidin-1-yl-carbonyl)-brombenzol

Hergestellt analog Beispiel 1b aus 4-Brom-3,5-dimethyl-benzoesäure, Pyrrolidin, O-(Benzotriazol-1-yl)-N,N, N',N'-tetramethyluroniumtetrafluorborat und Triethylamin in Dimethylformamid.
Ausbeute: 63 % der Theorie,
$R_f$-Wert: 0.45 (Kieselgel; Methylenchlorid/Ethanol = 19:1)

b. 2,5-Dimethyl-4-(pyrrolidin-1-yl-carbonyl)-benzylanilin

2.3 g (0.01 Mol) 2,5-Dimethyl-4-(pyrrolidin-1-yl-carbonyl)-brombenzol und 1.3 g (0.012 Mol) Benzylamin werden in 25 ml Toluol gelöst und nach Zugabe von 4.6 g Cäsiumcarbonat, 100 mg Palladium-II-acetat und 200 mg 2,2'-Bis(-diphenylphosphino)-1,1'-binaphthyl 7 Stunden unter Argonatmosphäre bei 100°C gerührt. Nach dem Abkühlen wird mit Eiswasser verdünnt und mit Essigester extrahiert. Die vereinigten organischen Extrakte werden getrocknet und eingedampft. Der Rückstand wird an Kieselgel chromatographiert, wobei mit Methylenchlorid/Ethanol (50:1 und 25:1) eluiert wird.
Ausbeute: 60 % der Theorie,
$R_f$-Wert: 0.30 (Kieselgel; Methylenchlorid/Ethanol = 9:1)

c. 2,5-Dimethyl-4-(pyrrolidin-1-yl-carbonyl)-anilin

Hergestellt analog Beispiel 1c aus 2,5-Dimethyl-4-(pyrrolidin-1-yl-carbonyl)-benzylanilin und Palladium auf Aktivkohle in Methanol.
Ausbeute: 94 % der Theorie,
$R_f$-Wert: 0.30 (Kieselgel; Essigester/Petrolether = 1:1)

d. 2-Benzyloxy-5-brom-phenylessigsäure

Eine Lösung von 12.4 g (0.053 Mol) 2-Hydroxy-5-brom-phenylessigsäure in 125 ml Dimethylformamid wird mit 14 g (0.125 Mol) Kalium-tert.butylat versetzt. Nach 15 Minuten bei Raumtemperatur werden 18.5 g (0.108 Mol) Benzylbromid zugegeben. Die Reaktionslösung wird 3 Stunden bei Raumtemperatur gerührt, auf Eiswasser gegossen und mit Essigester extrahiert. Die vereinten organischen Extrakte werden getrocknet und eingedampft. Der Rückstand wird in 100 ml Ethanol gelöst und nach Zusatz von 50 ml 2N Natronlauge 3 Stunden bei Raumtemperatur gerührt. Das Solvens wird abdestilliert, der Rückstand wird mit 2N Salzsäure auf pH 4 gestellt. Nach Extraktion mit Essigester werden die organischen Phasen getrocknet und eingedampft. Der Rückstand wird an Kieselgel chromatographiert und mit Petrolether/Essigester (8:2) eluiert.
Ausbeute: 6.7 g (38 % der Theorie),
$R_f$-Wert: 0.50 (Kieselgel; Essigester/Petrolether = 1:1)

e. 2-Benzyloxy-5-cyano-phenylessigsäure

Hergestellt analog Beispiel 2d aus 2-Benzyloxy-5-brom-phenylessigsäure, Kupfer-I-cyanid, Tetrakis-triphenylphosphin-palladium-(0) und Aluminiumoxid in Dimethylformamid.
Ausbeute: 26 % der Theorie,

$R_f$-Wert: 0.45 (Kieselgel; Methylenchlorid/Ethanol = 19:1)

f. 2-(5-Cyano-2-benzyloxy-phenyl)-N-[2,5-dimethyl-4-(pyrrolidin-1-yl-carbonyl)-phenyl]-acetamid

Hergestellt analog Beispiel 1b aus 2-Benzyloxy-5-cyano-phenylessigsäure, 2,5-Dimethyl-4-(pyrrolidin-1-yl-carbonyl)-anilin, O-(Benzotriazol-1-yl)-N,N,N',N'-tetramethyluroniumtetrafluorborat und N-Methylmorpholin in Tetrahydrofuran.
Ausbeute: 44 % der Theorie,
$R_f$-Wert: 0.75 (Kieselgel; Essigester/Ethanol = 9:1)

g. 2-(5-Carbamimidoyl-2-benzyloxy-phenyl)-N-[2,5-dimethyl-4-(pyrrolidin-1-yl-carbonyl)-phenyl]-acetamid

Hergestellt analog Beispiel 1e aus 2-(5-Cyano-2-benzyloxyphenyl)-N-[2,5-dimethyl-4-(pyrrolidin-1-yl-carbonyl)-phenyl]-acetamid und Salzsäure/Ammoniumcarbonat in Ethanol.
Ausbeute: 86 % der Theorie,
$R_f$-Wert: 0.20 (Kieselgel; Methylenchlorid/Ethanol/Eisessig = 8:2:0.01)

h. 2-(5-Carbamimidoyl-2-hydroxy-phenyl)-N-[2,5-dimethyl-4-(pyrrolidin-1-yl-carbonyl)-phenyl]-acetamid-hydrochlorid

355 mg (0.68 mMol) 2-(5-Carbamimidoyl-2-benzyloxy-phenyl)-N-[2,5-dimethyl-4-(pyrrolidin-1-yl-carbonyl)-phenyl]-acetamid werden in 40 ml Methanol gelöst und nach Zugabe von 250 mg Palladium auf Aktivkohle 15 Minuten mit Wasserstoff hydriert. Anschließend wird vom Katalysator abfiltriert und eingedampft.
Ausbeute: 145 mg (49 % der Theorie),
$R_f$-Wert: 0.10 (Kieselgel; Methylenchlorid/Ethanol/Eisessig = 8:2:0.01)
$C_{22}H_{26}N_4O_3$ x HCl (394.48/430.94)
Massenspektrum: $(M+H)^+$ = 395
$(M-H)^-$ = 393

Analog Beispiel 5 werden folgende Verbindungen hergestellt:

(1) 2-(5-Carbamimidoyl-2-hydroxy-phenyl)-N-[3-methyl-4-(piperidin-1-yl-carbonyl)-phenyl]-acetamid-hydrochlorid
Ausbeute: 98% der Theorie,
$R_f$-Wert: 0.75 (Reversed Phase RP8; 5%ige Kochsalzlösung/Methanol = 1:4)
$C_{22}H_{26}N_4O_3$ x HCl (394.49/430.94)
Massenspektrum: $M^+$ = 395
$(M+Cl)^-$ = 429/431 (Cl)
$(M-H)^-$ = 393

(2) 2-(5-Carbamimidoyl-2-hydroxy-phenyl)-N-[3-methyl-4-(2-methyl-pyrrolidin-1-yl-carbonyl)-phenyl]-acetamid-hydrochlorid Ausbeute: 100% der Theorie,
$R_f$-Wert: 0.7 (Reversed Phase RP8; 5%ige Kochsalzlösung/Methanol = 1:4)
$C_{22}H_{26}N_4O_3$ x HCl (394.49/430.94)
Massenspektrum: $M^+$ = 395
$(M+Cl)^-$ = 429/431 (Cl)
$(M-H)^-$ = 393

Beispiel 6

2-(2-Benzyloxy-5-carbamimidoyl-phenyl)-N-(2-ethoxycarbonylethyl)-N-[3-methyl-4-(pyrrolidin-1-yl-carbonyl)-phenyl]-acetamid-hydrochlorid

a. N-(2-Methoxycarbonyl-ethyl)-3-methyl-4-(pyrrolidin-1-ylcarbonyl)-anilin

1.5 g (7.3 mMol) 3-Methyl-4-(pyrrolidin-1-yl-carbonyl)-anilin, 20 ml (220 mMol) Acrylsäuremethylester, 1 ml (2.2 mMol) Triton B und 60 mg (0.27 mMol) 2,5-Di-tert.butyl-hydrochinon werden 22 Stunden bei 85°C gerührt. Anschließend wird das Reaktionsgemisch eingedampft, der Rückstand wird an Kieselgel chromatographiert, wobei mit Methylenchlorid + 0 bis 5 % Ethanol eluiert wird.
Ausbeute: 1.6 g (76 % der Theorie),
$R_f$-Wert: 0.70 (Kieselgel; Methylenchlorid/Ethanol = 9:1)

b. 2-(2-Benzyloxy-5-cyano-phenyl)-N-(2-ethoxycarbonyl-ethyl)-N-[3-methyl-4-(pyrrolidin-1-yl-carbonyl)-phenyl]-acetamid

0.8 g (2.88 mMol) N-(2-Methoxycarbonyl-ethyl)-3-methyl-4-(pyrrolidin-1-yl-carbonyl)-anilin werden in 50 ml Tetrahydrofuran gelöst und nach Zugabe von 1.1 ml (7.86 mMol) Triethylamin und 0.8 g (2.62 mMol) 2-Benzyloxy-5-cyano-phenylessigsäurechlorid 8 Stunden bei Raumtemperatur gerührt. Anschließend wird mit Wasser verdünnt und mit Methylenchlorid extrahiert. Die vereinigten organischen Extrakte werden getrocknet und eingedampft. Der Rückstand wird an Kieselgel chromatographiert, wobei mit Methylenchlorid eluiert wird.
Ausbeute: 1.0 g (71 % der Theorie),
$R_f$-Wert: 0.72 (Kieselgel; Methylenchlorid/Ethanol = 9:1)

c. 2-(2-Benzyloxy-5-carbamimidoyl-phenyl)-N-(2-ethoxycarbonylethyl)-N-[3-methyl-4-(pyrrolidin-1-yl-carbonyl)-phenyl]-acetamid-hydrochlorid

Hergestellt analog Beispiel 1e aus 2-(2-Benzyloxy-5-cyanophenyl) -N-(2-ethoxycarbonyl-ethyl)-N- [3-methyl-4-(pyrrolidin-1-yl-carbonyl)-phenyl]-acetamid und Salzsäure/Ammoniumcarbonat in Ethanol.
Ausbeute: 44 % der Theorie,
$R_f$-Wert: 0.17 (Kieselgel; Methylenchlorid/Ethanol = 4:1) $C_{33}H_{38}N_4O_5$ x HCl (570.69/607.16)
Massenspektrum: $(M+H)^+$ = 571

Beispiel 7

2-(2-Hydroxy-5-carbamimidoyl-phenyl)-N-(2-ethoxycarbonylethyl)-N-[3-methyl-4-(pyrrolidin-1-yl-carbonyl)-phenyl]-acetamid-hydrochlorid

Hergestellt analog Beispiel 5h aus 2-(2-Benzyloxy-5-carbamimidoyl-phenyl)-N-(2-ethoxycarbonyl-ethyl) -N-[3-methyl-4-(pyrrolidin-1-yl-carbonyl)-phenyl]-acetamid-hydrochlorid und Palladium auf Aktivkohle in Methanol.
Ausbeute: 96 % der Theorie,
$R_f$-Wert: 0.45 (Reversed Phase RP 8; Methanol/5%ige Natriumchlorid-Lösung = 6:4)
$C_{26}H_{32}N_4O_5$ x HCl (480.57/517.04)
Massenspektrum: $(M+H)^+$ = 481

Beispiel 8

2-(2-Hydroxy-5-carbamimidoyl-phenyl)-N-(2-carboxy-ethyl)-N-[3-methyl-4-(pyrrolidin-1-yl-carbonyl)-phenyl]-acetamidhydrochlorid

0.3 g (0.58 mMol) 2-(2-Hydroxy-5-carbamimidoyl-phenyl)-N-(2-ethoxycarbonyl-ethyl)-N-[3-methyl-4-(pyrrolidin-1-ylcarbonyl)-phenyl]-acetamid-hydrochlorid werden in einer Mischung von 3.2 ml (3.2 mMol) 1-molarer Lithiumhydroxidlösung, 6.2 ml Wasser und 7.6 ml Tetrahydrofuran 2 Stunden bei Raumtemperatur gerührt. Nach Zusatz von 74 mg Ammoniumchlorid wird die Lösung eingedampft. Der Rückstand wird an Reversed Phase chromatographiert und mit Wasser eluiert.
Ausbeute: 0.2 g (71 % der Theorie),
$R_f$-Wert: 0.62 (Reversed Phase RP 8; Methanol/5%ige Natriumchlorid-Lösung = 6:4)
$C_{24}H_{28}N_4O_5$ x HCl (452.52/488.97)
Massenspektrum: $(M+H)^+$ = 453
$(M-H)^-$ = 451

Analog Beispiel 8 werden folgende Verbindungen hergestellt:

(1) 2-(5-Carbamimidoyl-2-hydroxy-phenyl)-N-[3-methyl-4-(N-(3-carboxypropionyl)-N-cyclopentyl-amino)-phenyl]-acetamid-hydrochlorid
Ausbeute: 83% der Theorie,
$C_{25}H_{30}N_4O_5$ x HCl (466.55/503.01)
$R_f$-Wert: 0.84 (Reversed Phase RP8; 5%ige Kochsalzlösung/Methanol = 6:4)

(2) 2-(5-Carbamimidoyl-2-hydroxy-phenyl)-N-[3-methyl-4-(N-carboxyacetyl-N-cyclopentyl-amino)-phenyl]-acetamidhydrochlorid

Referenz beispiel 9

3-Carbamimidoyl-N-[4-(pyrrolidin-3-yl-oxy)-benzyl]-benzamiddihydrochlorid

a. N-tert.Butyloxycarbonyl-3-pyrrolidinol

5.8 g (66.5 mMol) 3-Pyrrolidinol und 6.7 g (67 mMol) Triethylamin werden in 80 ml Methylenchlorid gelöst und tropfenweise mit einer Lösung von 15.3 g (70 mMol) Di-tert.Butyl-dicarbonat in 40 ml Methylenchlorid versetzt. Nach 16 Stunden bei Raumtemperatur wird mit Wasser verrührt, die organische Phase wird getrocknet und eingedampft.
Ausbeute: 12.4 g (100 % der Theorie),
$R_f$-Wert: 0.75 (Kieselgel; Essigester/Methanol = 9:1)

b. 4-[(N-tert.Butyloxycarbonyl)-pyrrolidin-3-yl-oxy]-benzonitril

3.8 g (20 mMol) N-tert.Butyloxycarbonyl-3-pyrrolidinol werden in 100 ml Tetrahydrofuran gelöst und nach Zugabe von 2.4 g (20 mMol) 4-Hydroxybenzonitril, 5.7 g (22 mMol) Triphenylphosphin und 3.9 g (22 mMol) Diethylazodicarbonsäurediethylester 18 Stunden bei Raumtemperatur gerührt. Das Lösungsmittel wird abdestilliert und der Rückstand an Kieselgel chromatographiert, wobei mit Cyclohexan/Essigester (10:5) eluiert wird.
Ausbeute: 4.5 g (78 % der Theorie),
$R_f$-Wert: 0.40 (Kieselgel; Cyclohexan/Essigester = 10:5)

c. 4-[(N-tert.Butyloxycarbonyl)-pyrrolidin-3-yl-oxy]-benzylamin

4.5.g (15.6 mMol) 4-[(N-tert.Butyloxycarbonyl)-pyrrolidin-3-yl-oxy]-benzonitril werden in 100 ml Methanol und 50 ml methanolischem Ammoniak gelöst und nach Zugabe von 1 g Raney-Nickel 2 Stunden bei 50°C mit Wasserstoff hydriert. Anschließend wird vom Katalysator abfiltriert und eingedampft.
Ausbeute: 4.2 g (92 % der Theorie),
$R_f$-Wert: 0.08 (Kieselgel; Essigester/Methanol = 4:1)

d. 3-Cyano-N-[4-(N'-tert.butyloxycarbonyl-pyrrolidin-3-yloxy)-benzyl]-benzamid

1.1 g (3.8 mMol) 4-[(N-tert.Butyloxycarbonyl)-pyrrolidin-3-yloxy]-benzylamin werden in 30 ml Methylenchlorid gelöst und nach Zugabe von 0.9 g (9 mMol) Triethylamin portionsweise mit 1.6 g (3.8 mMol) 3-Cyanobenzoesäurechlorid versetzt. Nach 4 Stunden bei Raumtemperatur wird die Lösung mit Wasser versetzt, die organische Phase wird getrocknet und eingedampft.
Ausbeute: 1.5 g (94 % der Theorie),
$R_f$-Wert: 0.27 (Kieselgel; Methylenchlorid/Essigester = 9:1)

e. 3-Carbamimidoyl-N-[4-(pyrrolidin-3-yl-oxy)-benzyl]-benzamid-dihydrochlorid

Hergestellt analog Beispiel 1e aus 3-Cyano-N-[4-(N'-tert.butyloxycarbonyl-pyrrolidin-3-yl-oxy)-benzyl]-benzamid und Salzsäure/Ammoniumchlorid in Ethanol.
Ausbeute: 100 % der Theorie,
Schmelzpunkt: ab 180°C (Zersetzung)
$C_{19}H_{22}N_4O_2$ x 2 HCl (338.41/411.41)
Massenspektrum: $(M+H)^+$ = 339

Referenz beispiel 10

3-Carbamimidoyl-N-(4-dimethylaminomethyl-benzyl)-benzamid-dihydrochlorid

a. 4-Cyano-N,N-dimethyl-benzylamin

Zu einer Lösung von 10 g (0.05 Mol) 4-Cyanobenzylbromid in 400 ml Ether wird bei -5°C eine Lösung von 7.3 g (0.16 Mol) Dimethylamin in 100 ml Ether zugetropft. Anschließend wird das Reaktionsgemisch 2 Stunden bei -5°C und 20 Stunden bei Raumtemperatur gerührt. Nach Zusatz von 200 ml Wasser und 200 ml konz. Salzsäure wird die wässrige Lösung abgetrennt, mit Natronlauge alkalisch gestellt und mit Methylenchlorid extrahiert. Die vereinigten

organische Extrakte werden getrocknet und eingedampft.
Ausbeute: 8 g (100 % der Theorie),
$R_f$-Wert: 0.58 (Kieselgel; Methylenchlorid/Ethanol = 9:1)

b. 4-Dimethylaminomethyl-benzylamin

Hergestellt analog Beispiel 9c aus 4-Cyano-N.N-dimethylbenzylamin, methanolischem Ammoniak und Raney-Nickel/Wasserstoff.
Ausbeute: 94 % der Theorie,
$R_f$-Wert: 0.13 (Kieselgel; Methylenchlorid/Ethanol = 9:1)

c. 3-Cyano-N-(4-dimethylaminomethyl-benzyl)-benzamid

Hergestellt analolg Beispiel 9d aus 4-Dimethylaminomethyl-benzylamin, 3-Cyanobenzoylchlorid und Triethylamin in Methylenchlorid.
Ausbeute: 73 % der Theorie,
Schmelzpunkt: 100°C

d. 3-Carbamimidoyl-N-(4-dimethylaminomethyl-benzyl)-benzamiddihydrochlorid

Hergestellt analog Beispiel 1e aus 3-Cyano-N-(4-dimethylaminomethyl-benzyl)-benzamid und Salzsäure/Ammoniumcarbonat in Ethanol.
Ausbeute: 100 % der Theorie,
Schmelzpunkt: ab 101°C (Zersetzung)
$C_{18}H_{22}N_4O$ x 2 HCl (310.40/383.40)
Massenspektrum: $(M+H)^+$ = 311
Analog Referenz beispiel 10 werden folgende Verbindungen hergestellt:

(1) 2- (5-Carbamimidoyl-2-hydroxy-phenyl) -N- [3-methyl-4-(pyrrolidin-1-yl-methyl)-phenyl]-acetamid-dihydrochlorid
Ausbeute: 60% d. Theorie
Rf-Wert (Reversed Phase RP8; 5% Kochsalzlösung/Methanol = 2:3): 0.7
$C_{21}H_{26}N_4O_2$ x 2 HCl (366.47/439.38)
Massenspektrum: (M+H)+ =367
(M-H)-=365

(2) 2-(5-Carbamimidoyl-2-hydroxy-phenyl)-N-[3-methyl-4-(imidazol-1-yl-methyl)-phenyl]-acetamid-dihydrochlorid
Ausbeute: 57% d. Theorie
Rf-Wert (Reversed Phase RP8; 5% Kochsalzlösung/Methanol = 2:3): 0.7
$C_{20}H_{21}N_5O_2$ x 2 HCl (363.42/436.33)
Massenspektrum: (M+H)+ =364
(M-H)-=362

(3) 2-(5-Carbamimidoyl-2-hydroxy-phenyl)-N-[3-methyl-4-(2-methyl-imidazol-1-yl-methyl)-phenyl]-acetamid-dihydrochlorid

Beispiel 11

2-(5-Carbamimidoyl-2-hydroxy-phenyl)-N-[3-methyl-4-(2-aminosulfonyl-phenyl)-phenyl]-acetamid-hydrochlorid

a. 3-Allyl-4-hydroxy-benzonitril

82.3 g (0.52 Mol) 4-Allyloxy-benzonitril werden unter Stickstoffatmosphäre 2 Stunden auf 200°C erhitzt. Nach Abkühlung wird das Rohprodukt an Kieselgel gereinigt, wobei anfangs mit Petrolether, später mit Petrolether/Essigester (9:1, 8:2, 7:3 und 1:1) eluiert wird. Die einheitlichen Fraktionen werden vereinigt und eingedampft, der Rückstand wird mit Petrolether gewaschen und getrocknet.
Ausbeute: 43 g (52% der Theorie),
$R_f$-Wert: 0.45 (Kieselgel; Petrolether/Essigester = 1:1)

$C_{10}H_9NO$ (159.19)
Massenspektrum: $(M-H)^-$ = 158
$(2M+Na)^+$ = 341

b. 3-Allyl-4-benzyloxy-benzonitril

Hergestellt analog Beispiel 5d aus 3-Allyl-4-hydroxy-benzonitril und Benzylbromid/Kaliumcarbonat in Dimethylformamid.
Ausbeute: 90% der Theorie,
Schmelzpunkt: 59-60°C
$R_f$-Wert: 0.55 (Kieselgel; Petrolether/Essigester = 4:1)

c. 2-Benzyloxy-5-cyano-phenylessigsäure

30 g (0.12 Mol) 3-Allyl-4-benzyloxy-benzonitril werden in 450 ml Acetonitril gelöst und bei 40°C mit 0.7 g Rutheniumtrichlorid-hydrat und einer Lösung von 179.7 g (0.84 Mol) Natriumperjodat in 1 Liter Wasser versetzt. Nach beendeter Zugabe wird das Reaktionsgemisch noch weitere 30 Minuten auf 40°C erwärmt und anschließend 3 x mit je 1 Liter Essigester extrahiert. Die organischen Phasen werden mit Kochsalzlösung gewaschen und über Natriumsulfat getrocknet. Das Rohprodukt wird unter Zusatz von Aktivkohle aus Petrolether/Essigester (7:3) umkristallisiert
Ausbeute: 18.4 g (58% der Theorie),
Schmelzpunkt: 144-145°C
$R_f$-Wert: 0.2 (Kieselgel; Petrolether/Essigester = 1:1)
$C_{16}H_{13}NO_3$ (267.29)
Massenspektrum: $(M-H)^-$ = 266
$(M+Na)^+$ = 290

d. 2-(5-Cyano-2-benzyloxy-phenyl)-N-[3-methyl-4-(2-tert.butylaminosulfonyl-phenyl)-phenyl]-acetamid

Hergestellt analog Beispiel 1b aus 2-Benzyloxy-5-cyano-phenylessigsäure und 4'-Amino-2'-methyl-biphenyl-2-sulfonsäuretert.-butylamid/O-(Benzotriazol-1-yl)-N,N,N',N'-tetramethyluroniumtetrafluorborat/Triethylamin in Dimethylformamid.
Ausbeute: 60% der Theorie,
$R_f$-Wert: 0.5 (Kieselgel; Methylenchlorid/Ethanol = 19:1)

e. 2-(5-Carbamimidoyl-2-benzyloxy-phenyl)-N-[3-methyl-4-(2-aminosulfonyl-phenyl)-phenyl]-acetamid-hydrochlorid

Hergestellt analog Beispiel 1e aus 2-(5-Cyano-2-benzyloxyphenyl)-N-[3-methyl-4-(2-tert.butylaminosulfonyl-phenyl)-phenyl]-acetamid und Salzsäure/Ammoniumcarbonat in Ethanol.
Ausbeute: 70% der Theorie,
$R_f$-Wert: 0.3 (Kieselgel; Methylenchlorid/Ethanol = 9:1 + 1% Eisessig)
$C_{29}H_{28}N_4O_4S$ x HCl (528.63/565.08)
Massenspektrum: $(M-H)^-$ = 527
$(M+H)^+$ = 529

f. 2-(5-Carbamimidoyl-2-hydroxy-phenyl)-N-[3-methyl-4-(2-aminosulfonyl-phenyl)-phenyl]-acetamid-hydrochlorid

Hergestellt analog Beispiel 5h aus 2-(5-Carbamimidoyl-2-benzyloxy-phenyl)-N-[3-methyl-4-(2-aminosulfonyl-phenyl)-phenyl]-acetamid-hydrochlorid und Wasserstoff/Palladium auf Aktivkohle
Ausbeute: 62% der Theorie,
$R_f$-Wert: 0.45 (Reversed Phase RP8; 5%ige Kochsalzlösung/Methanol = 1:1)
$C_{22}H_{22}N_4O_4S$ x HCl (438.52/474.97)
Massenspektrum: $(M+H)^+$ = 439
$(M+Cl)^-$ = 473/5 (Cl)

Analog Beispiel 11 werden folgende Verbindungen hergestellt:

(1) 2-(5-Carbamimidoyl-2-hydroxy-phenyl)-N-(3-methyl-4-phenylphenyl]-acetamid-hydrochlorid
Ausbeute: 13% der Theorie,
$R_f$-Wert: 0.15 (Kieselgel; Methylenchlorid/Ethanol = 4:1) $C_{22}H_{21}N_3O_2$ x HCl (359.45/395.9)

Massenspektrum: $(M+H)^+$ = 360
$(M-H)^-$ = 358

(2) 2-(5-Carbamimidoyl-2-hydroxy-phenyl)-N-[3-methyl-4-(2-aminosulfonyl-5-methyl-phenyl)-phenyl]-acetamid-hydrochlorid
Ausbeute: 23% d. Theorie
Rf-Wert (Kieselgel; Methylenchlorid/Ethanol = 7:3): 0.3 $C_{23}H_{24}N_4O_4S$ x HCl (452.54/488.99)
Massenspektrum: (M+H)+ =453
(M-H)-=451

Beispiel 12

2-[5-(N-Benzoyl-carbamimidoyl)-2-hydroxy-phenyl]-N-[3-methyl-4-(pyrrolidin-1-yl-carbonyl)-phenyl]-acetamid

a. 2-[5-(N-Benzoyl-carbamimidoyl)-2-benzyloxy-phenyl]-N-[3-methyl-4-(pyrrolidin-1-yl-carbonyl)-phenyl]-acetamid

350 mg (0.69 mMol) 2-(5-Carbamimidoyl-2-benzyloxy-phenyl)-N-[3-methyl-4-(pyrrolidin-1-yl-carbonyl)-phenyl]-acetamidhydrochlorid werden in 40 ml Methylenchlorid suspendiert und mit 1.0 ml Triethylamin und 300 mg (1.3 mMol) 4-Nitrophenylbenzoat versetzt. Das Reaktionsgemisch wird 4 Stunden unter Rückfluß erhitzt. Nach Zugabe von 100 ml Kochsalzlösung wird die wässrige Phase 3 x mit Methylenchlorid extrahiert. Die vereinigten organischen Phasen werden über Natriumsulfat getrocknet und eingeengt. Das Rohprodukt wird an Kieselgel gereinigt, wobei anfangs mit Methylenchlorid, später mit Methylenchlorid/Ethanol (50:1 und 19:1) eluiert wird. Die einheitlichen Fraktionen werden vereinigt, eingeengt und mit Petrolether/Ether (1:1) verrührt. Der gebildete Feststoff wird abgesaugt und getrocknet.
Ausbeute: 280 mg (71% der Theorie),
$R_f$-Wert: 0.2 (Kieselgel; Petrolether/Essigester = 1:1)
$C_{35}H_{34}N_4O_4$ (574.69)
Massenspektrum: $(M-H)^-$ = 573
$(M+H)^+$ = 575

b. 2- [5-(N-Benzoyl-carbamimidoyl)-2-hydroxy-phenyl]-N-[3-methyl-4-(pyrrolidin-1-yl-carbonyl)-phenyl]-acetamid

Hergestellt analog Beispiel 5h aus 2-[5-(N-Benzoyl-carbamimidoyl)-2-benzyloxy-phenyl]-N-[3-methyl-4-(pyrrolidin-1-yl-carbonyl)-phenyl]-acetamid und Wasserstoff/Palladium auf Aktivkohle.
Ausbeute: 31% der Theorie,
$R_f$-Wert: 0.3 (Kieselgel; Methylenchlorid/Ethanol = 19:1)
$C_{28}H_{28}N_4O_4$ (484.56)
Massenspektrum: $(M+H)^+$ = 485
$(M+Na)^+$ = 507

Analog Beispiel 12 werden folgende Verbindungen hergestellt:

(1) 2-[5-(N-n-Hexyloxycarbonyl-carbamimidoyl)-2-hydroxy-phenyl)-N-[3-methyl-4-(pyrrolidin-1-yl-carbonyl) -phenyl]-acetamid
Ausbeute: 17% der Theorie,
$R_f$-Wert: 0.3 (Kieselgel; Methylenchlorid/Ethanol = 4:1)
$C_{28}H_{36}N_4O_5$ (508.62)
Massenspektrum: $(M+H)^+$ = 509
$(M-H)^-$ = 507

(2) 2-[5-(N-Benzoyl-carbamimidoyl)-2-methoxy-phenyl]-N-[3-methyl-4-(pyrrolidin-1-yl-carbonyl)-phenyl]-acetamid
Ausbeute: 40% der Theorie,
$R_f$-Wert: 0.3 (Kieselgel; Methylenchlorid/Ethanol = 19:1)
$C_{29}H_{30}N_4O_4$ (498.59)
Massenspektrum: $(M+H)^+$ = 499
$(M-H)^-$ = 497

(3) 2-[5-(N-n-Hexyloxycarbonyl-carbamimidoyl)-2-methoxy-phenyl]-N-[3-methyl-4-(pyrrolidin-1-yl-carbonyl)-phenyl]-acetamid

Ausbeute: 35% der Theorie,
$R_f$-Wert: 0.25 (Kieselgel; Methylenchlorid/Ethanol = 19:1)
$C_{29}H_{28}N_4O_5$ (522.65)
Massenspektrum: $(M+H)^+ = 523$
$(M-H)^- = 521$
$(M+Na)^+ = 545$

(4) 2-[5-(N-Ethyloxycarbonyl-carbamimidoyl)-2-methoxy-phenyl]-N-[3-methyl-4-(pyrrolidin-1-yl-carbonyl)-phenyl]-acetamid
Ausbeute: 32% der Theorie,
$R_f$-Wert: 0.45 (Kieselgel; Methylenchlorid/Ethanol = 9:1)
$C_{25}H_{30}N_4O_5$ (466.54)
Massenspektrum: $(M+H)^+ = 467$
$(M-H)^- = 465$
$(M+Na)^+ = 489$

Beispiel 13

2-[5-(N-Hydroxy-carbamimidoyl)-2-hydroxy-phenyl]-N-[3-methyl-4-(pyrrolidin-1-yl-carbonyl)-phenyl]-acetamid-acetat

a. 2-[5-(N-Hydroxy-carbamimidoyl)-2-benzyloxy-phenyl]-N-[3-methyl-4-(pyrrolidin-1-yl-carbonyl)-phenyl]-acetamidacetat

1.1 g (2.5 mMol) 2-(5-Cyano-2-benzyloxy-phenyl)-N-[3-methyl-4-(pyrrolidin-1-yl-carbonyl)-phenyl]-acetamid werden in 100 ml Methanol gelöst und mit einer Lösung von 300 mg (5 mMol) Hydroxylamin-hydrochlorid in 2.0 ml Wasser versetzt. Nach Zugabe von 800 mg (2.5 mMol) Cäsiumcarbonat und 300 mg (3.0 mMol) Natriumcarbonat wird das Reaktionsgemisch 6 Stunden unter Rückfluß erhitzt. Nach Abkühlung und Zusatz von 0.5 l Eiswasser wird das gebildete Rohprodukt abgesaugt und an Kieselgel gereinigt, wobei anfangs mit Methylenchlorid und Methylenchlorid/Ethanol (19:1), später mit Methylenchlorid/Ethanol (9:1 + 1% Eisessig und 4:1 + 1% Eisessig) eluiert wird. Die einheitlichen Fraktionen werden vereinigt und eingeengt.
Ausbeute: 620 mg (51% der Theorie),
$R_f$-Wert: 0.3 (Kieselgel; Methylenchlorid/Ethanol = 9:1): 0.3
$C_{28}H_{30}N_4O_4$ (486.58)
Massenspektrum: $(M-H)^- = 485$
$(M+H)^+ = 487$
$(M+Na)^+ = 509$

b. 2-[5-(N-Hydroxy-carbamimidoyl)-2-hydroxy-phenyl]-N-[3-methyl-4-(pyrrolidin-1-yl-carbonyl)-phenyl]-acetamid-acetat

Hergestellt analog Beispiel 5h aus 2-[5-(N-Hydroxy-carbamimidoyl)-2-benzyloxy-phenyl]-N-[3-methyl-4-(pyrrolidin-1-yl-carbonyl)-phenyl]-acetamid-acetat und Wasserstoff/Palladium auf Aktivkohle.
Ausbeute: 50% der Theorie,
$R_f$-Wert: 0.25 (Kieselgel; Methylenchlorid/Ethanol = 4:1 + 1% Eisessig)
$C_{21}H_{24}N_4O_4 \times CH_3COOH$ (396.45/456.5)
Massenspektrum: $(M+H)^+ = 397$
$(M-H)^- = 395$

Analog Beispiel 13 wird folgende Verbindung hergestellt:

(1) 2-[5-(N-Hydroxy-carbamimidoyl)-2-methoxy-phenyl]-N-[3-methyl-4-(pyrrolidin-1-yl-carbonyl)-phenyl]-acetamid-acetat
Ausbeute: 7% der Theorie,
$R_f$-Wert: 0.28 (Kieselgel; Methylenchlorid/Ethanol = 4:1 + 1% Eisessig)
$C_{22}H_{26}N_4O_4$ x CH3COOH (410.48/470.52)
Massenspektrum: $(M+H)^+ = 411$
$(M-H)^- = 409$
$(M+Na)^+ = 433$

Beispiel 14

Trockenampulle mit 75 mg Wirkstoff pro 10 ml

Zusammensetzung:

| | |
|---|---|
| Wirkstoff | 75,0 mg |
| Mannitol | 50,0 mg |
| Wasser für Injektionszwecke | ad 10,0 ml |

Herstellung:

Wirkstoff und Mannitol werden in Wasser gelöst. Nach Abfüllung wird gefriergetrocknet. Die Auflösung zur gebrauchsfertigen Lösung erfolgt mit Wasser für Injektionszwecke.

Beispiel 15

Trockenampulle mit 35 mg Wirkstoff pro 2 ml

Zusammensetzung:

| | |
|---|---|
| Wirkstoff | 35,0 mg |
| Mannitol | 100,0 mg |
| Wasser für Injektionszwecke | ad 2,0 ml |

Herstellung:

Wirkstoff und Mannitol werden in Wasser gelöst. Nach Abfüllung wird gefriergetrocknet.
Die Auflösung zur gebrauchsfertigen Lösung erfolgt mit Wasser für Injektionszwecke.

Beispiel 16

Tablette mit 50 mg Wirkstoff

Zusammensetzung:

| | |
|---|---|
| (1) Wirkstoff | 50,0 mg |
| (2) Milchzucker | 98,0 mg |
| (3) Maisstärke | . 50,0 mg |
| (4) Polyvinylpyrrolidon | 15,0 mg |
| (5) Magnesiumstearat | 2,0 mg |
| | 215,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe. Durchmesser der Tabletten: 9 mm.

Beispiel 17

Tablette mit 350 mg Wirkstoff

Zusammensetzung:

| | |
|---|---|
| (1) Wirkstoff | 350,0 mg |
| (2) Milchzucker | 136,0 mg |
| (3) Maisstärke | 80,0 mg |
| (4) Polyvinylpyrrolidon | 30,0 mg |
| (5) Magnesiumstearat | 4,0 mg |
| | 600,0 mg |

Herstellung:

(1), (2) und (3) werden gemischt und mit einer wäßrigen Lösung von (4) granuliert. Dem getrockneten Granulat wird (5) zugemischt. Aus dieser Mischung werden Tabletten gepreßt, biplan mit beidseitiger Facette und einseitiger Teilkerbe.
Durchmesser der Tabletten: 12 mm.

Beispiel 18

Kapseln mit 50 mg Wirkstoff

Zusammensetzung:

| | |
|---|---|
| (1) Wirkstoff | 50,0 mg |
| (2) Maisstärke getrocknet | 58,0 mg |
| (3) Milchzucker pulverisiert | 50,0 mg |
| (4) Magnesiumstearat | 2,0 mg |
| | 160,0 mg |

Herstellung:

(1) wird mit (3) verrieben. Diese Verreibung wird der Mischung aus (2) und (4) unter intensiver Mischung zugegeben.
Diese Pulvermischung wird auf einer Kapselabfüllmaschine in Hartgelatine-Steckkapseln Größe 3 abgefüllt.

Beispiel 19

Kapseln mit 350 mg Wirkstoff

Zusammensetzung:

| | |
|---|---|
| (1) Wirkstoff | 350,0 mg |
| (2) Maisstärke getrocknet | 46,0 mg |
| (3) Milchzucker pulverisiert | 30,0 mg |
| (4) Magnesiumstearat | 4,0 mg |
| | 430,0 mg |

Herstellung:

(1) wird mit (3) verrieben. Diese Verreibung wird der Mischung aus (2) und (4) unter intensiver Mischung zugegeben.

Diese Pulvermischung wird auf einer Kapselabfüllmaschine in Hartgelatine-Steckkapseln Größe 0 abgefüllt.

Beispiel 20

Suppositorien mit 100 mg Wirkstoff

| 1 Zäpfchen enthält: | |
|---|---|
| Wirkstoff | 100,0 mg |
| Polyethylenglykol (M.G. 1500) | 600,0 mg |
| Polyethylenglykol (M.G. 6000) | 460,0 mg |
| Polyethylensorbitanmonostearat | 840,0 mg |
| | 2 000,0 mg |

Herstellung:

Das Polyethylenglykol wird zusammen mit Polyethylensorbitanmonostearat geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

**Patentansprüche**

1. Carbonsäureamide der allgemeinen Formel

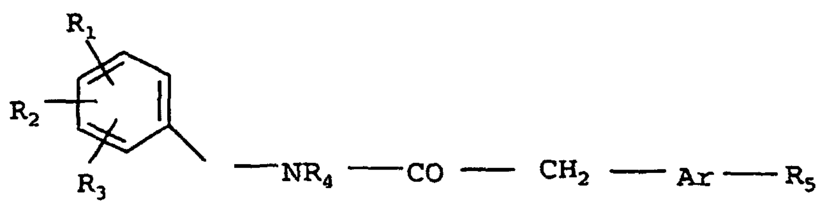

(I),

in der
Ar eine gegebenenfalls durch ein Fluor-, Chlor- oder Bromatom, durch eine Trifluormethyl-, $C_{1-3}$-Alkyl-, Hydroxy-, $C_{1-3}$-Alkoxy-, Phenyl-$C_{1-3}$-alkoxy-, Amino-, $C_{1-3}$-Alkylamino- oder Di-($C_{1-3}$-Alkyl)-aminogruppe substituierte Phenylen- oder Naphthylengruppe, wobei die Phenylengruppe durch ein weiteres Fluor-, Chloroder Bromatom oder durch eine weitere $C_{1-3}$-Alkylgruppe substituiert sein kann,
eine Thienylen-, Thiazolylen-, Pyridinylen-, Pyrimidinylen-, Pyrazinylen- oder Pyridazinylengruppe, die im Kohlenstoffgerüst gegebenenfalls durch eine $C_{1-3}$-Alkylgruppe substituiert ist,
$R_1$ eine gegebenenfalls durch eine Amino-, $C_{1-3}$-Alkylamino-, Di-($C_{1-3}$-Alkyl)-amino-, Phenyl-, Naphthyl-, Heteroaryl- oder 4- bis 7-gliedrige Cycloalkyleniminogruppe substituierte $C_{1-3}$-Alkylgruppe,
eine $C_{3-7}$-Cycloalkylgruppe, die in 1-Stellung durch eine 5- bis 7-gliedrige Cycloalkyleniminocarbonylgruppe substituiert ist, eine Amino-, $C_{1-5}$-Alkylamino-, $C_{5-7}$-Cycloalkylamino- oder Phenyl-$C_{1-3}$-alkylaminogruppe, die jeweils am Aminstickstoffatom durch eine Benzoyl- oder Phenylsulfonylgruppe oder durch eine gegebenenfalls im $C_{1-3}$-Alkylteil durch eine Carboxygruppe substituierte $C_{1-3}$-Alkyl- oder $C_{1-3}$-Alkylcarbonylgruppe substituiert sein kann,
eine gegebenenfalls durch eine $C_{1-3}$-Alkylgruppe substituierte 4-bis 7-gliedrige Cycloalkyleniminocarbonyl- oder Cycloalkyleniminosulfonylgruppe,
eine gegebenenfalls durch eine oder zwei $C_{1-3}$-Alkylgruppen substituierte Aminosulfonylgruppe,
eine gegebenenfalls durch ein Fluor-, Chlor- oder Bromatom, durch eine Trifluormethyl-, Aminosulfonyl-, $C_{1-3}$-

Alkyl- oder $C_{1-3}$-Alkoxygruppe substituierte Phenylgruppe, die zusätzlich durch ein Fluor-, Chlor- oder Bromatom, durch eine Trifluormethyl-, $C_{1-3}$-Alkyl- oder $C_{1-3}$-Alkoxygruppe substituiert sein kann,

eine $C_{1-3}$-Alkoxy-, Phenyl-$C_{1-3}$-alkoxy-, Heteroaryloxy- oder Heteroaryloxy-$C_{1-3}$-alkoxygruppe, in der der Alkoxyteil jeweils in 2- oder 3-Stellung auch durch eine Amino-, $C_{1-3}$-Alkylaminooder Di-($C_{1-3}$-Alkyl)-aminogruppe substituiert sein kann,

eine $C_{3-7}$-Cycloalkoxygruppe, wobei die Methylengruppe in 3- oder 4-Stellung in einer $C_{5-7}$-Cycloalkoxygruppe durch eine -NH-Gruppe ersetzt sein kann, wobei die -NH-Gruppe

durch eine $C_{1-3}$-Alkylgruppe, die in 2- oder 3-Stellung durch eine Amino-, $C_{1-3}$-Alkylamino- oder Di-($C_{1-3}$-Alkyl)-aminogruppe substituiert sein kann, durch eine $C_{1-3}$-Alkylcarbonyl-, Arylcarbonyl- oder Arylsulfonylgruppe oder

durch Aminocarbonyl-, $C_{1-3}$-Alkylaminocarbonyl- oder Di-($C_{1-3}$-Alkyl)-aminocarbonylgruppe, in denen jeweils das Sauerstoffatom der Carbonylgruppe durch eine Iminogruppe ersetzt ist, substituiert sein kann,

$R_2$ ein Wasserstoff-, Fluor-, Chlor- oder Bromatom, eine $C_{1-3}$-Alkyl-, Hydroxy- oder $C_{1-3}$-Alkoxygruppe,

$R_3$ ein Wasserstoffatom oder eine $C_{1-3}$-Alkylgruppe,

$R_4$ ein Wasserstoffatom oder eine gegebenenfalls durch eine Carboxygruppe substituierte $C_{1-3}$-Alkylgruppe und

$R_5$ eine Cyanogruppe oder eine gegebenenfalls durch eine oder zwei $C_{1-3}$-Alkylgruppen substituierte Amidinogruppe bedeuten, wobei

unter den vorstehend erwähnten Heteroarylgruppen eine gegebenenfalls durch eine $C_{1-3}$-Alkylgruppe substituierte 5-gliedrige Heteroarylgruppe, die im heteroaromatischen Teil

eine gegebenenfalls durch eine $C_{1-3}$-Alkylgruppe substituierte Iminogruppe, ein Sauerstoff- oder Schwefelatom,

eine gegebenenfalls durch eine $C_{1-3}$-Alkylgruppe substituierte Iminogruppe und ein Sauerstoff-, Schwefel- oder Stickstoffatom,

eine gegebenenfalls durch eine $C_{1-3}$-Alkylgruppe substituierte Iminogruppe und zwei Stickstoffatome oder ein Sauerstoff- oder Schwefelatom und zwei Stickstoffatome enthält,

oder eine gegebenenfalls durch eine $C_{1-3}$-Alkylgruppe substituierte 6-gliedrige Heteroarylengruppe, die im heteroaromatischen Teil

ein oder zwei Stickstoffatome enthält,

zu verstehen ist,

die bei der Definition der vorstehend erwähnten Resten erwähnten Carboxygruppen durch eine in-vivo in eine Carboxygruppe überführbare Gruppe oder durch eine unter physiologischen Bedingungen negativ geladene Gruppe ersetzt sein und

die bei der Definition der vorstehend erwähnten Resten erwähnten Amino- und Iminogruppen durch einen in vivo abspaltbaren Rest substituiert sein können, wobei

unter einer in-vivo in eine Carboxygruppe überführbaren Gruppe beispielsweise eine Hydroxmethylgruppe, eine mit einem Alkohol veresterte Carboxygruppe, in der der alkoholische Teil vorzugsweise ein $C_{1-6}$-Alkanol, ein Phenyl-$C_{1-3}$-alkanol, ein $C_{3-9}$-Cycloalkanol, wobei ein $C_{5-8}$-Cycloalkanol zusätzlich durch eine oder zwei $C_{1-3}$-Alkylgruppen substituiert sein kann, ein $C_{5-8}$-Cycloalkanol, in dem eine Methylengruppe in 3- oder 4-Stellung durch ein Sauerstoffatom oder durch eine gegebenenfalls durch eine $C_{1-3}$-Alkyl-, Phenyl-$C_{1-3}$-alkyl-, Phenyl-$C_{1-3}$-alkoxycarbonyl- oder $C_{2-6}$-Alkanoylgruppe substituierte Iminogruppe ersetzt ist und der Cycloalkanolteil zusätzlich durch eine oder zwei $C_{1-3}$-Alkylgruppen substituiert sein kann, ein $C_{4-7}$-Cycloalkenol, ein $C_{3-5}$-Alkenol, ein Phenyl-$C_{3-5}$-alkenol, ein $C_{3-5}$-Alkinol oder Phenyl-$C_{3-5}$-alkinol mit der Maßgabe, daß keine Bindung an das Sauerstoffatom von einem Kohlenstoffatom ausgeht, welches eine Doppel- oder Dreifachbindung trägt, ein $C_{3-8}$-Cycloalkyl-$C_{1-3}$-alkanol, ein Bicycloalkanol mit insgesamt 8 bis 10 Kohlenstoffatomen, das im Bicycloalkylteil zusätzlich durch eine oder zwei $C_{1-3}$-Alkylgruppen substituiert sein kann, ein 1,3-Dihydro-3-oxo-1-isobenzfuranol oder ein Alkohol der Formel

$$R_a\text{-CO-O-}(R_bCR_c)\text{-OH},$$

verstanden wird, in dem

$R_a$ eine $C_{1-8}$-Alkyl-, $C_{5-7}$-Cycloalkyl-, Phenyl- oder Phenyl-$C_{1-3}$-alkylgruppe,

$R_b$ ein Wasserstoffatom, eine $C_{1-3}$-Alkyl-, $C_{5-7}$-Cycloalkyl- oder Phenylgruppe und

$R_c$ ein Wasserstoffatom oder eine $C_{1-3}$-Alkylgruppe darstellen,

unter einer unter physiologischen Bedingungen negativ geladenen Gruppe eine Tetrazol-5-yl-, Phenylcarbonylaminocarbonyl-, Trifluormethylcarbonylaminocarbonyl-, $C_{1-6}$-Alkylsulfonylamino-, Phenylsulfonylamino-, Benzylsulfonylamino-, Trifluormethylsulfonylamino-, $C_{1-6}$-Alkylsulfonylaminocarbonyl-, Phenylsulfonylaminocarbonyl-, Benzylsulfonylaminocarbonyl- oder Perfluor-$C_{1-6}$-alkylsulfonylaminocarbonylgruppe

und unter einem von einer Imino- oder Aminogruppe in-vivo abspaltbaren Rest beispielsweise eine Hydroxygruppe, eine Acylgruppe wie eine gegebenenfalls durch Fluor-, Chlor-, Bromoder Jodatome, durch $C_{1-3}$-Alkyl- oder $C_{1-3}$-Alkoxygruppen monooder disubstituierte Benzoylgruppe, wobei die Substituenten gleich oder verschieden sein können, eine Pyridinoylgruppe oder eine $C_{1-16}$-Alkanoylgruppe wie die Formyl-, Acetyl-, Propionyl-, Butanoyl-, Pentanoyl- oder Hexanoylgruppe, eine 3,3,3-Trichlorpropionyl- oder Allyloxycarbonylgruppe, eine $C_{1-16}$-Alkoxycarbonyl- oder $C_{1-16}$-Alkylcarbonyloxygruppe, in denen Wasserstoffatome ganz oder teilweise durch Fluor- oder Chloratome ersetzt sein können, wie die Methoxycarbonyl-, Ethoxycarbonyl-, Propoxycarbonyl-, Isopropoxycarbonyl-, Butoxycarbonyl-, tert. Butoxycarbonyl-, Pentoxycarbonyl-, Hexoxycarbonyl-, Octyloxycarbonyl-, Nonyloxycarbonyl-, Decyloxycarbonyl-, Undecyloxycarbonyl-, Dodecyloxycarbonyl-, Hexadecyloxycarbonyl-, Methylcarbonyloxy-, Ethylcarbonyloxy-, 2,2,2-Trichlorethylcarbonyloxy-, Propylcarbonyloxy-, Isopropylcarbonyloxy-, Butylcarbonyloxy-, tert.Butylcarbonyloxy-, Pentylcarbonyloxy-, Hexylcarbonyloxy-, Octylcarbonyloxy-, Nonylcarbonyloxy-, Decylcarbonyloxy-, Undecylcarbonyloxy-, Dodecylcarbonyloxy- oder Hexadecylcarbonyloxygruppe, eine Phenyl-$C_{1-6}$-alkoxycarbonylgruppe wie die Benzyloxycarbonyl-, Phenylethoxycarbonyl- oder Phenylpropoxycarbonylgruppe, eine 3-Amino-propionylgruppe, in der die Aminogruppe durch $C_{1-6}$-Alkyl- oder $C_{3-7}$-Cycloalkylgruppen mono- oder disubstituiert und die Substituenten gleich oder verschieden sein können, eine $C_{1-3}$-Alkylsulfonyl-$C_{2-4}$-alkoxycarbonyl-, $C_{1-3}$-Alkoxy-$C_{2-4}$-alkoxy-$C_{2-4}$-alkoxycarbonyl-, $R_a$-CO-O-($R_b$C$R_c$)-O-CO-, $C_{1-6}$-Alkyl-CO-NH-($R_d$C$R_e$)-O-CO- oder $C_{1-6}$-Alkyl-CO-O-($R_d$C$R_e$)-($R_d$C$R_e$)-O-CO-Gruppe, in denen $R_a$ bis $R_c$ wie vorstehend erwähnt definiert sind und
$R_d$ und $R_e$, die gleich oder verschieden sein können, Wasserstoffatome oder $C_{1-3}$-Alkylgruppen darstellen, verstanden werden,
deren Isomere und deren Salze.

**2.** Verbindungen der allgemeinen Formel I gemäß Anspruch 1, in denen
Ar eine gegebenenfalls durch ein Fluor-, Chlor- oder Bromatom, durch eine Methyl-, Hydroxy-, Methoxy- oder Benzyloxygruppe substitiuierte Phenylengruppe, welche durch eine weitere Methylgruppe substituiert sein kann,
$R_1$ eine gegebenenfalls durch ein Fluor-, Chlor- oder Bromatom, durch eine Trifluormethyl-, Aminosulfonyl-, $C_{1-3}$-Alkyl- oder $C_{1-3}$-Alkoxygruppe substituierte Phenylgruppe, die zusätzlich durch ein Fluor-, Chlor- oder Bromatom, durch eine Trifluormethyl-, $C_{1-3}$-Alkyl- oder $C_{1-3}$-Alkoxygruppe substituiert sein kann,
eine durch eine Dimethylamino-, Pyrrolidino- oder Imidazolylgruppe substituierte Methylgruppe, wobei der Imidazolylteil durch eine Methylgruppe substituiert sein kann,
eine Amino-, $C_{1-5}$-Alkylamino-, Cyclopentylamino- oder Benzylaminogruppe, die am Aminstickstoffatom durch eine Carboxy-$C_{1-2}$-alkyl-, $C_{1-3}$-Alkoxycarbonyl-$C_{1-2}$-alkyl-, Carboxy-$C_{1-2}$-alkylcarbonyl- oder $C_{1-3}$-Alkoxycarbonyl-$C_{1-2}$-alkylcarbonylgruppe substituiert sein kann,
eine Benzoylamino- oder Phenylsulfonylaminogruppe,
eine Cyclopropylgruppe, die in 1-Stellung durch eine 5- bis 7-gliedrige Cycloalkyleniminocarbonylgruppe substituiert ist,
eine gegebenenfalls durch eine Methylgruppe substituierte Pyrrolidinocarbonyl-, Piperidinocarbonyl-, Pyrrolidinosulfonyloder Piperidinosulfonylgruppe,
eine $C_{1-3}$-Alkoxygruppe, in der der Alkoxyteil jeweils in 2- oder 3-Stellung durch eine Amino-, $C_{1-3}$-Alkylamino- oder Di-($C_{1-3}$-Alkyl)-aminogruppe substituiert sein kann,
eine Phenyl-$C_{1-3}$-alkoxy- oder Pyridinyloxygruppe,
eine $C_{5-7}$-Cycloalkoxygruppe, in der die Methylengruppe in 3-oder 4-Stellung durch eine -NH-Gruppe ersetzt sein kann, wobei die -NH-Gruppe
durch eine $C_{1-3}$-Alkyl- oder $C_{2-3}$-Alkanoylgruppe,
durch eine $C_{2-3}$-Alkanoyl- oder Aminocarbonylgruppe, in der jeweils das Sauerstoffatom der Carbonylgruppe durch eine Iminogruppe ersetzt ist, substituiert sein kann,
$R_2$ ein Wasserstoff-, Fluor-, Chlor- oder Bromatom, eine Methyl-, Hydroxy- oder Methoxygruppe,
$R_3$ ein Wasserstoffatom oder eine Methylgruppe,
$R_4$ ein Wasserstoffatom oder eine gegebenenfalls durch eine Carboxy- oder $C_{1-3}$-Alkoxycarbonylgruppe substituierte Methyl- oder Ethylgruppe und
$R_5$ eine Cyanogruppe oder eine gegebenenfalls durch eine $C_{1-6}$-Alkoxycarbonyl- oder Benzoylgruppe substituierte Amidinogruppe bedeuten,
deren Isomere und deren Salze.

**3.** Verbindungen der allgemeinen Formel I gemäß Anspruch 1, in denen
Ar eine gegebenenfalls durch eine Methyl-, Hydroxy-, Methoxyoder Benzyloxygruppe substitiuierte Phenylengruppe,
$R_1$ eine gegebenenfalls durch ein Fluor-, Chlor- oder Bromatom, durch eine Trifluormethyl-, Aminosulfonyl-, $C_{1-3}$-

Alkyl- oder $C_{1-3}$-Alkoxygruppe substituierte Phenylgruppe, die zusätzlich durch ein Fluor-, Chlor- oder Bromatom, durch eine Trifluormethyl-, $C_{1-3}$-Alkyl- oder $C_{1-3}$-Alkoxygruppe substituiert sein kann,
eine Cyclopropylgruppe, die in 1-Stellung durch eine 5- bis 7-gliedrige Cycloalkyleniminocarbonylgruppe substituiert ist, oder eine 4- bis 7-gliedrige Cycloalkyleniminocarbonylgruppe,
eine gegebenenfalls durch eine Methylgruppe substituierte Pyrrolidinocarbonyl-, Piperidinocarbonyl- oder Pyrrolidinosulfonylgruppe,
$R_2$ ein Wasserstoff-, Fluor-, Chlor- oder Bromatom oder eine Methylgruppe,
$R_3$ ein Wasserstoffatom oder eine Methylgruppe,
$R_4$ ein Wasserstoffmatom oder eine durch eine Carboxy-, Methoxycarbonyl- oder Ethoxycarbonylgruppe substituierte Methyloder Ethylgruppe und
$R_5$ eine gegebenenfalls durch eine $C_{1-6}$-Alkoxycarbonyl- oder Benzoylgruppe substituierte Amidinogruppe bedeuten,
deren Isomere und deren Salze.

**4.** Folgende Verbindungen der allgemeinen Formel I gemäß Anspruch 1:

(a) 2-(5-Carbamimidoyl-2-hydroxy-phenyl)-N-[3-methyl-4-(pyrrolidin-1-yl-carbonyl)-phenyl]-acetamid,

(b) 2-(2-Benzyloxy-5-carbamimidoyl-phenyl)-N-(2-ethoxycarbonyl-ethyl)-N-[3-methyl-4-(pyrrolidin-1-yl-carbonyl)-phenyl]-acetamid,

(c) 2-(2-Hydroxy-5-carbamimidoyl-phenyl)-N-(2-ethoxycarbonylethyl)-N-[3-methyl-4-(pyrrolidin-1-yl-carbonyl)-phenyl]-acetamid,

(d) 2-(2-Hydroxy-5-carbamimidoyl-phenyl)-N-(2-carboxy-ethyl)-N-[3-methyl-4-(pyrrolidin-1-yl-carbonyl)-phenyl]-acetamid,

(e) 2-(5-Carbamimidoyl-2-hydroxy-phenyl)-N-[3-methyl-4-(piperidin-1-yl-carbonyl)-phenyl]-acetamid und

(f) 2-(5-Carbamimidoyl-2-hydroxy-phenyl)-N-[3-methyl-4-(2-aminosulfonyl-phenyl)-phenyl]-acetamid,

in denen die Amidinogruppe zusätzlich durch eine $C_{1-6}$-Alkoxycarbonyl- oder Benzoylgruppe substituiert sein kann, und deren Salze.

**5.** 2-(5-Carbamimidoyl-2-hydroxy-phenyl)-N-[3-methyl-4-(pyrrolidin-1-yl-carbonyl)-phenyl]-acetamid und dessen Salze.

**6.** Physiologisch verträgliche Salze der Verbindungen gemäß den Ansprüchen 1 bis 5, in denen $R_5$ eine der in den Ansprüchen 1 bis 5 erwähnten Amidinogruppen darstellt.

**7.** Arzneimittel, enthaltend eine Verbindung nach mindestens einem der Ansprüche 1 bis 5, in denen $R_5$ eine der in den Ansprüchen 1 bis 5 erwähnten Amidinogruppen darstellt, oder ein Salz gemäß Anspruch 6 neben gegebenenfalls einem oder mehreren inerten Trägerstoffen und/oder Verdünnungsmitteln.

**8.** Verwendung einer Verbindung nach mindestens einem der Ansprüche 1 bis 5, in denen $R_5$ eine der in den Ansprüchen 1 bis 5 erwähnten Amidinogruppen darstellt, oder ein Salz gemäß Anspruch 6 zur Herstellung eines Arzneimittels mit einer antithrombotischen Wirkung.

**9.** Verfahren zur Herstellung eines Arzneimittels gemäß Anspruch 7, **dadurch gekennzeichnet, daß** auf nichtchemischem Wege eine Verbindung nach mindestens einem der Ansprüche 1 bis 5, in denen $R_5$ eine der in den Ansprüchen 1 bis 5 erwähnten Amidinogruppen darstellt, oder ein Salz gemäß Anspruch 6 in einen oder mehrere inerte Trägerstoffe und/oder Verdünnungsmittel eingearbeitet wird.

**10.** Verfahren zur Herstellung der Verbindungen gemäß den Ansprüchen 1 bis 6, **dadurch gekennzeichnet, daß**

a) eine Verbindung der allgemeinen Formel

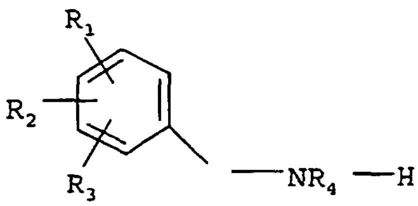

(II),

in der
$R_1$ bis $R_4$ wie in den Ansprüchen 1 bis 5 erwähnt definiert sind, mit einer Carbonsäure der allgemeinen Formel

$$HO—CO—CH_2—Ar—R_5 \qquad (III),$$

in der
Ar, und $R_5$ wie in den Ansprüchen 1 bis 5 erwähnt definiert sind, oder mit deren reaktionsfähigen Derivaten acyliert wird oder

b) zur Herstellung einer Verbindung der allgemeinen Formel I, in der $R_5$ eine Amidinogruppe, die durch eine oder zwei $C_{1-3}$-Alkylgruppen substituiert sein kann, eine gegebenenfalls im Reaktionsgemisch gebildete Verbindung der allgemeinen Formel

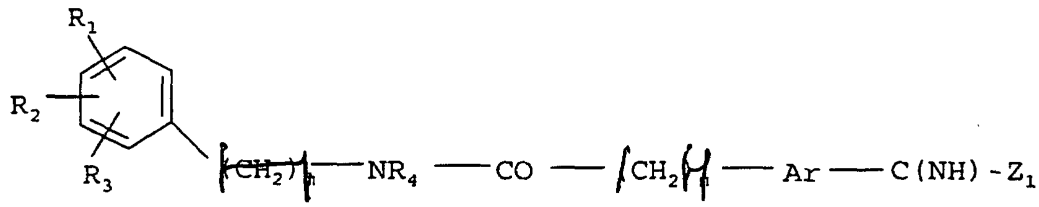

(IV),

in der
$R_1$ bis $R_4$, und Ar wie in den Ansprüchen 1 bis 5 erwähnt definiert sind und
$Z_1$ eine Alkoxy-, Aralkoxy-, Alkylthio- oder Aralkylthiogruppe darstellt, mit einem Amin der allgemeinen Formel

$$H - R_6NR_7 , \qquad (V)$$

in der
$R_6$ und $R_7$, die gleich oder verschieden sein können, jeweils ein Wasserstoffatom oder eine $C_{1-3}$-Alkylgruppe bedeuten, oder mit dessen Salzen umgesetzt wird und
gewünschtenfalls anschließend eine so erhaltene Verbindung der allgemeinen Formel I, die eine Amino- oder Iminogruppe enthält, mittels einem entsprechenden Acylderivat in eine entsprechende Acylverbindung der allgemeinen Formel I übergeführt wird und/oder
eine so erhaltene Verbindung der allgemeinen Formel I, die eine veresterte Carboxygruppe enthält, mittels Hydrolyse in eine entsprechende Carbonsäure der allgemeinen Formel I übergeführt wird und/oder
eine so erhaltene Verbindung der allgemeinen Formel I, die eine Carboxygruppe enthält, mittels Veresterung in einen entsprechenden Ester übergeführt wird und/oder
ein während den Umsetzungen zum Schütze von reaktiven Gruppen verwendeter Schutzrest abgespalten wird und/oder
eine so erhaltene Verbindung der allgemeinen Formel I in ihre Stereoisomere aufgetrennt wird und/oder
eine so erhaltene Verbindung der allgemeinen Formel I in ihre Salze, insbesondere für die pharmazeutische Anwendung in ihre physiologisch verträglichen Salze mit einer anorganischen oder organischen Säure oder Base, übergeführt wird.

## Claims

1. Carboxylic acid amides of general formula

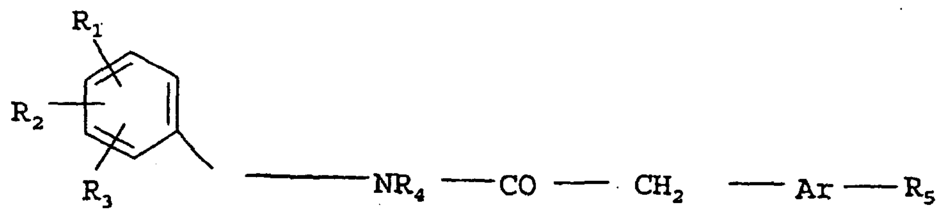

(I),

wherein

Ar denotes a phenylene or naphthylene group optionally substituted by a fluorine, chlorine or bromine atom, by a trifluoromethyl, $C_{1-3}$-alkyl, hydroxy, $C_{1-3}$-alkoxy, phenyl-$C_{1-3}$-alkoxy, amino, $C_{1-3}$-alkylamino or di-($C_{1-3}$-alkyl)-amino group, whilst the phenylene group may be substituted by another fluorine, chlorine or bromine atom or by another $C_{1-3}$-alkyl group,

a thienylene, thiazolylene, pyridinylene, pyrimidinylene, pyrazinylene or pyridazinylene group optionally substituted in the carbon skeleton by a $C_{1-3}$-alkyl group,

$R_1$ denotes a $C_{1-3}$-alkyl group optionally substituted by an amino, $C_{1-3}$-alkylamino, di-($C_{1-3}$-alkyl)-amino, phenyl, naphthyl, heteroaryl or 4- to 7-membered cycloalkyleneimino group,

a $C_{3-7}$-cycloalkyl group which is substituted in the 1 position by a 5- to 7-membered cycloalkyleneiminocarbonyl group,

an amino, $C_{1-5}$-alkylamino, $C_{5-7}$-cycloalkylamino or phenyl-$C_{1-3}$-alkylamino group which may in each case be substituted at the amino-nitrogen atom by a benzoyl or phenylsulphonyl group or by a $C_{1-3}$-alkyl or $C_{1-3}$-alkylcarbonyl group optionally substituted in the $C_{1-3}$-alkyl moiety by a carboxy group,

a 4- to 7-membered cycloalkyleneiminocarbonyl or cycloalkyleneiminosulphonyl group optionally substituted by a $C_{1-3}$-alkyl group,

an aminosulphonyl group optionally substituted by one or two $C_{1-3}$-alkyl groups,

a phenyl group optionally substituted by a fluorine, chlorine or bromine atom, by a trifluoromethyl, aminosulphonyl, $C_{1-3}$-alkyl or $C_{1-3}$-alkoxy group, which may additionally be substituted by a fluorine, chlorine or bromine atom or by a trifluoromethyl, $C_{1-3}$-alkyl or $C_{1-3}$-alkoxy group,

a $C_{1-3}$-alkoxy, phenyl-$C_{1-3}$-alkoxy, heteroaryloxy or heteroaryloxy-$C_{1-3}$-alkoxy group wherein the alkoxy moiety may also be substituted in the 2 or 3 position in each case by an amino, $C_{1-3}$-alkylamino or di-($C_{1-3}$-alkyl)-amino group,

a $C_{3-7}$-cycloalkoxy group, whilst the methylene group in the 3 or 4 position in a $C_{5-7}$-cycloalkoxy group may be replaced by an -NH- group, whilst the -NH- group may be substituted

by a $C_{1-3}$-alkyl group which may be substituted in the 2 or 3 position by an amino, $C_{1-3}$-alkylamino or di-($C_{1-3}$-alkyl)-amino group, by a $C_{1-3}$-alkylcarbonyl, arylcarbonyl or arylsulphonyl group or

by an aminocarbonyl, $C_{1-3}$-alkylaminocarbonyl or di-($C_{1-3}$-alkyl)-aminocarbonyl group, wherein in each case the oxygen atom of the carbonyl group is replaced by an imino group,

$R_2$ denotes a hydrogen, fluorine, chlorine or bromine atom, a $C_{1-3}$-alkyl, hydroxy or $C_{1-3}$-alkoxy group,

$R_3$ denotes a hydrogen atom or a $C_{1-3}$-alkyl group,

$R_4$ denotes a hydrogen atom or a $C_{1-3}$-alkyl group optionally substituted by a carboxy group and

$R_5$ denotes a cyano group or an amidino group optionally substituted by one or two $C_{1-3}$-alkyl groups, whilst

by the abovementioned heteroaryl groups is meant a 5-membered heteroaryl group optionally substituted by a $C_{1-3}$-alkyl group which contains, in the heteroaromatic moiety,

an imino group optionally substituted by a $C_{1-3}$-alkyl group, or an oxygen or sulphur atom,

an imino group optionally substituted by a $C_{1-3}$-alkyl group and an oxygen, sulphur or nitrogen atom,

an imino group optionally substituted by a $C_{1-3}$-alkyl group and two nitrogen atoms or

an oxygen or sulphur atom and two nitrogen atoms,

or a 6-membered heteroarylene group optionally substituted by a $C_{1-3}$-alkyl group which contains one or two nitrogen atoms in the heteroaromatic moiety,

the carboxy groups mentioned in the definition of the abovementioned groups may be replaced by a group which may be converted into a carboxy group *in vivo* or by a group which is negatively charged under physiological conditions and

the amino and imino groups mentioned in the definition of the abovementioned groups may be replaced by a group which may be cleaved *in vivo*, while

by a group which can be converted *in vivo* into a carboxy group is meant, for example, a hydroxymethyl group, a carboxy group esterified with an alcohol, wherein the alcoholic moiety preferably denotes a $C_{1-6}$-alkanol, a phenyl-$C_{1-3}$-alkanol, a $C_{3-9}$-cycloalkanol, whilst a $C_{5-8}$-cycloalkanol may additionally be substituted by one or two $C_{1-3}$-alkyl groups, a $C_{5-8}$-cycloalkanol wherein a methylene group in the 3 or 4 position is replaced by an oxygen atom or by an imino group optionally substituted by a $C_{1-3}$-alkyl, phenyl-$C_{1-3}$-alkyl, phenyl-$C_{1-3}$-alkoxycarbonyl or $C_{2-6}$-alkanoyl group and the cycloalkanol moiety may additionally be substituted by one or two $C_{1-3}$-alkyl groups, a $C_{4-7}$-cycloalkenol, a $C_{3-5}$-alkenol, a phenyl-$C_{3-5}$-alkenol, a $C_{3-5}$-alkynol or phenyl-$C_{3-5}$-alkynol, with the proviso that no bond to the oxygen atom starts from a carbon atom which carries a double or triple bond, a $C_{3-8}$-cycloalkyl-$C_{1-3}$-alkanol, a bicycloalkanol having a total of 8 to 10 carbon atoms which may additionally be substituted by one or two $C_{1-3}$-alkyl groups in the bicycloalkyl moiety, a 1,3-dihydro-3-oxo-1-isobenzofuranol or an alcohol of formula

$$R_a\text{-CO-O-}(R_bCR_c)\text{-OH},$$

wherein

$R_a$ denotes a $C_{1-8}$-alkyl, $C_{5-7}$-cycloalkyl, phenyl or phenyl- $C_{1-3}$-alkyl group,

$R_b$ denotes a hydrogen atom, a $C_{1-3}$-alkyl, $C_{5-7}$-cycloalkyl or phenyl group and

$R_c$ denotes a hydrogen atom or a $C_{1-3}$-alkyl group,

by a group which is negatively charged under physiological conditions is meant a tetrazol-5-yl, phenylcarbonylaminocarbonyl, trifluoromethylcarbonylaminocarbonyl, $C_{1-6}$-alkylsulphonylamino, phenylsulphonylamino, benzylsulphonylamino, trifluoromethylsulphonylamino, $C_{1-6}$-alkylsulphonylaminocarbonyl, phenylsulphonylaminocarbonyl, benzylsulphonylaminocarbonyl or perfluoro-$C_{1-6}$-alkylsulphonylaminocarbonyl group

and by a group which can be cleaved *in vivo* from an imino or amino group is meant, for example, a hydroxy group, an acyl group such as a benzoyl group optionally mono- or disubstituted by fluorine, chlorine, bromine or iodine atoms or by $C_{1-3}$-alkyl or $C_{1-3}$-alkoxy groups, whilst the substituents may be identical or different, a pyridinoyl group or a $C_{1-16}$-alkanoyl group such as the formyl, acetyl, propionyl, butanoyl, pentanoyl or hexanoyl group, a 3,3,3-trichloropropionyl or allyloxycarbonyl group, a $C_{1-16}$-alkoxycarbonyl or $C_{1-16}$-alkylcarbonyloxy group wherein hydrogen atoms may be wholly or partially replaced by fluorine or chlorine atoms, such as the methoxycarbonyl, ethoxycarbonyl, propoxycarbonyl, isopropoxycarbonyl, butoxycarbonyl, tert. butoxycarbonyl, pentoxycarbonyl, hexoxycarbonyl, octyloxycarbonyl, nonyloxycarbonyl, decyloxycarbonyl, undecyloxycarbonyl, dodecyloxycarbonyl, hexadecyloxycarbonyl, methylcarbonyloxy, ethylcarbonyloxy, 2,2,2-trichloroethylcarbonyloxy, propylcarbonyloxy, isopropylcarbonyloxy, butylcarbonyloxy, tert.butylcarbonyloxy, pentylcarbonyloxy, hexylcarbonyloxy, octylcarbonyloxy, nonylcarbonyloxy, decylcarbonyloxy, undecylcarbonyloxy, dodecylcarbonyloxy or hexadecylcarbonyloxy group, a phenyl-$C_{1-6}$-alkoxycarbonyl group such as the benzyloxycarbonyl, phenylethoxycarbonyl or phenylpropoxycarbonyl group, a 3-amino-propionyl group wherein the amino group may be mono or disubstituted by $C_{1-6}$-alkyl or $C_{3-7}$-cycloalkyl groups and the substituents may be identical or different, a $C_{1-3}$-alkylsulphonyl-$C_{2-4}$-alkoxycarbonyl, $C_{1-3}$-alkoxy-$C_{2-4}$-alkoxy-$C_{2-4}$-alkoxycarbonyl, $R_a$-CO-O-($R_bCR_c$)-O-CO, $C_{1-6}$-alkyl-CO-NH-($R_dCR_e$)-O-CO or $C_{1-6}$-alkyl-CO-O-($R_dCR_e$)-($R_dCR_e$)-O-CO group wherein $R_a$ to $R_c$ are as hereinbefore defined and $R_d$ and $R_e$, which may be identical or different, denote hydrogen atoms or $C_{1-3}$-alkyl groups,

the isomers and salts thereof.

**2.** Compounds of general formula I according to claim 1, wherein

Ar denotes a phenylene group optionally substituted by a fluorine, chlorine or bromine atom or by a methyl, hydroxy, methoxy or benzyloxy group, which may be substituted by another methyl group,

$R_1$ denotes a phenyl group optionally substituted by a fluorine, chlorine or bromine atom or by a trifluoromethyl, aminosulphonyl, $C_{1-3}$-alkyl or $C_{1-3}$-alkoxy group, which may additionally be substituted by a fluorine, chlorine or bromine atom or by a trifluoromethyl, $C_{1-3}$-alkyl or $C_{1-3}$-alkoxy group,

a methyl group substituted by a dimethylamino, pyrrolidino or imidazolyl group, wherein the imidazolyl moiety may be substituted by a methyl group, an amino, $C_{1-5}$-alkylamino, cyclopentylamino or benzylamino group which may be substituted at the amino-nitrogen atom by a carboxy-$C_{1-2}$-alkyl, $C_{1-3}$-alkoxycarbonyl-$C_{1-2}$-alkyl, carboxy-$C_{1-2}$-alkylcarbonyl or $C_{1-3}$-alkoxycarbonyl-$C_{1-2}$-alkylcarbonyl group,

a benzoylamino or phenylsulphonylamino group,

a cyclopropyl group which is substituted in the 1 position by a 5- to 7-membered cycloalkyleneiminocarbonyl group, an optionally methyl-substituted pyrrolidinocarbonyl, piperidinocarbonyl, pyrrolidinosulphonyl or piperidinosulphonyl group,

a $C_{1-3}$-alkoxy group wherein the alkoxy moiety in the 2 or 3 position may be substituted in each case by an amino,

$C_{1-3}$-alkylamino or di-($C_{1-3}$-alkyl)-amino group,
a phenyl-$C_{1-3}$-alkoxy or pyridinyloxy group,
a $C_{5-7}$-cycloalkoxy group wherein the methylene group in the 3 or 4 position may be replaced by an -NH- group, whilst the -NH- group may be substituted
by a $C_{1-3}$-alkyl or $C_{2-3}$-alkanoyl group,
by a $C_{2-3}$-alkanoyl or aminocarbonyl group wherein in each case the oxygen atom of the carbonyl group is replaced by an imino group,
$R_2$ denotes a hydrogen, fluorine, chlorine or bromine atom, a methyl, hydroxy or methoxy group,
$R_3$ denotes a hydrogen atom or a methyl group,
$R_4$ denotes a hydrogen atom or a methyl or ethyl group optionally substituted by a carboxy or $C_{1-3}$-alkoxycarbonyl group and
$R_5$ denotes a cyano group or an amidino group optionally substituted by a $C_{1-6}$-alkoxycarbonyl or benzoyl group, the isomers thereof and the salts thereof.

**3.** Compounds of general formula I according to claim 1, wherein
Ar denotes a phenylene group optionally substituted by a methyl, hydroxy, methoxy or benzyloxy group,
$R_1$ denotes a phenyl group optionally substituted by a fluorine, chlorine or bromine atom or by a trifluoromethyl, aminosulphonyl, $C_{1-3}$-alkyl or $C_{1-3}$-alkoxy group, which may additionally be substituted by a fluorine, chlorine or bromine atom or by a trifluoromethyl, $C_{1-3}$-alkyl or $C_{1-3}$-alkoxy group,
a cyclopropyl group which is substituted in the 1 position by a 5- to 7-membered cycloalkyleneiminocarbonyl group, or a 4- to 7-membered cycloalkyleneiminocarbonyl group,
an optionally methyl-substituted pyrrolidinocarbonyl, piperidinocarbonyl or pyrrolidinosulphonyl group,
$R_2$ denotes a hydrogen, fluorine, chlorine or bromine atom or a methyl group,
$R_3$ denotes a hydrogen atom or a methyl group,
$R_4$ denotes a hydrogen atom or a methyl or ethyl group substituted by a carboxy, methoxycarbonyl or ethoxycarbonyl group and
$R_5$ denotes an amidino group optionally substituted by a $C_{1-6}$-alkoxycarbonyl or benzoyl group,
the isomers thereof and the salts thereof.

**4.** The following compounds of general formula I according to claim 1:

(a) 2-(5-carbamimidoyl-2-hydroxy-phenyl)-N-[3-methyl-4-(pyrrolidin-1-yl-carbonyl)-phenyl]-acetamide,

(b) 2-(2-benzyloxy-5-carbamimidoyl-phenyl)-N-(2-ethoxycarbonyl-ethyl)-N-[3-methyl-4-(pyrrolidin-1-yl-carbonyl)-phenyl]-acetamide,

(c) 2-(2-hydroxy-5-carbamimidoyl-phenyl)-N-(2-ethoxycarbonylethyl)-N-[3-methyl-4-(pyrrolidin-1-yl-carbonyl)-phenyl]-acetamide,

(d) 2-(2-hydroxy-5-carbamimidoyl-phenyl)-N-(2-carboxy-ethyl)-N-[3-methyl-4-(pyrrolidin-1-yl-carbonyl)-phenyl]-acetamide,

(e) 2-(5-carbamimidoyl-2-hydroxy-phenyl)-N-[3-methyl-4-(piperidin-1-yl-carbonyl)-phenyl]-acetamide and

(f) 2-(5-carbamimidoyl-2-hydroxy-phenyl)-N-[3-methyl-4-(2-aminosulphonyl-phenyl)-phenyl]-acetamide,

wherein the amidino group may additionally be substituted by a $C_{1-6}$-alkoxy-carbonyl or benzoyl group, and the salts thereof.

**5.** 2-(5-Carbamimidoyl-2-hydroxy-phenyl)-N-[3-methyl-4-(pyrrolidin-1-yl-carbonyl)-phenyl]-acetamide and the salts thereof.

**6.** Physiologically acceptable salts of the compounds according to claims 1 to 5 wherein $R_5$ denotes one of the amidino groups mentioned in claims 1 to 5.

**7.** Pharmaceutical compositions containing a compound according to at least one of claims 1 to 5, wherein $R_5$ denotes one of the amidino groups mentioned in claims 1 to 5, or a salt according to claim 6 optionally together with one or more inert carriers and/or diluents.

**8.** Use of a compound according to at least one of claims 1 to 5, wherein $R_5$ denotes one of the amidino groups mentioned in claims 1 to 5, or a salt according to claim 6, for preparing a pharmaceutical composition having an antithrombotic activity.

**9.** Process for preparing a pharmaceutical composition according to claim 7, **characterised in that** a compound according to at least one of claims 1 to 5, wherein $R_5$ denotes one of the amidino groups mentioned in claims 1 to 5, or a salt according to claim 6 is incorporated in one or more inert carriers and/or diluents by a non-chemical method.

**10.** Process for preparing the compounds according to claims 1 to 6, **characterised in that**

a) a compound of general formula

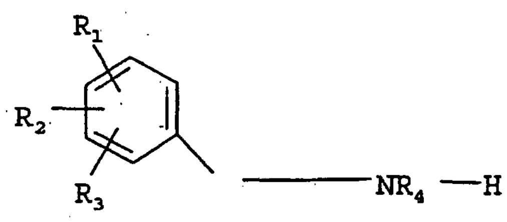

(II),

wherein
$R_1$ to $R_4$ are defined as in claims 1 to 5, is acylated with a carboxylic acid of general formula

$$HO—CO—CH_2—Ar—R_5 \qquad (III),$$

wherein
Ar and $R_5$ are defined as in claims 1 to 5, or with the reactive derivatives thereof, or

b) in order to prepare a compound of general formula I wherein $R_5$ denotes an amidino group which may be substituted by one or two $C_{1-3}$-alkyl groups; a compound of general formula

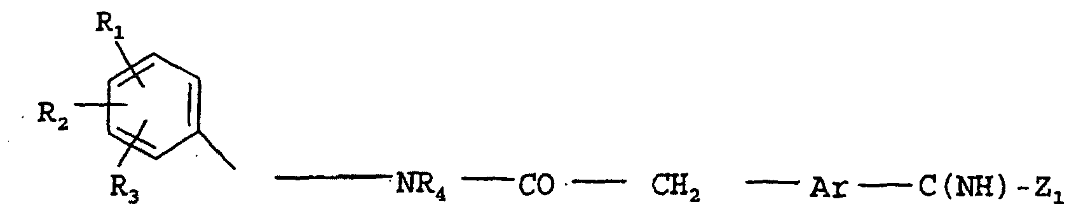

(IV),

optionally formed in the reaction mixture,
wherein
$R_1$ to $R_4$ and Ar are defined as in claims 1 to 5 and
$Z_1$ denotes an alkoxy, aralkoxy, alkylthio or aralkylthio group, is reacted with an amine of general formula

$$H - R_6NR_7 , \qquad (V)$$

wherein
$R_6$ and $R_7$, which may be identical or different, each denote a hydrogen atom or a $C_{1-3}$-alkyl group, or with the salts thereof, and
subsequently, if desired, a compound of general formula I thus obtained which contains an amino or imino group is converted by means of a corresponding acyl derivative into a corresponding acyl compound of general formula I and/or

a compound of general formula I thus obtained which contains an esterified carboxy group is converted by hydrolysis into a corresponding carboxylic acid of general formula I and/or
a compound of general formula I thus obtained which contains a carboxy group is converted by esterification into a corresponding ester and/or
a protecting group used to protect reactive groups during the reactions is cleaved and/or
a compound of general formula I thus obtained is resolved into the stereoisomers thereof, and/or
a compound of general formula I thus obtained is;converted into the salts thereof, particularly, for pharmaceutical use, into the physiologically acceptable salts thereof with an inorganic or organic acid or base.

## Revendications

**1.** Amides d'acide carboxylique de la formule générale :

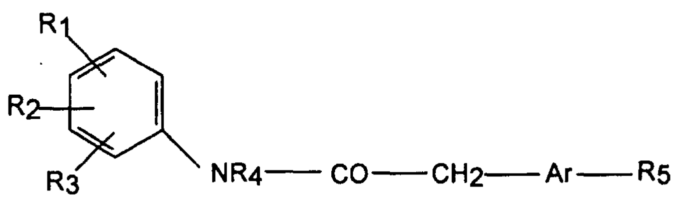

(I)

dans laquelle :

Ar représente un radical phénylène ou naphtylène, le cas échéant substitué par un atome de fluor, de chlore ou de brome, par un radical trifluorométhyle, alkyle en $C_{1-3}$, hydroxyle, alcoxy en $C_{1-3}$, phénylalcoxy en $C_{1-3}$, amino, (alkyl en $C_{1-3}$)amino ou di(alkyl en $C_{1-3}$)amino, où le radical phénylène peut être substitué par un autre atome de fluor, de chlore ou de brome ou par un autre radical alkyle en $C_{1-3}$,
représente un radical thiénylène, thiazolylène, pyridinylène, pyrimidinylène, pyrazinylène ou pyridazinylène, qui est le cas échéant, substitué sur le squelette carbone, par un radical alkyle en $C_{1-3}$ ;
$R_1$ représente un radical alkyle en $C_{1-3}$ le cas échéant substitué par un radical amino, (alkyl en $C_{1-3}$)amino, di (alkyl en $C_{1-3}$)amino, phényle, naphtyle, hétéroaryle ou cycloalkylèneimino ayant 4 à 7 membres,
représente un radical cycloalkyle en $C_{3-7}$, qui est substitué en position 1 par un radical cycloalkylèneiminocarbonyle ayant 5 à 7 membres,
représente un radical amino, (alkyl en $C_{1-5}$)amino, (cycloalkyl en $C_{5-7}$)amino ou phényl (alkyl en $C_{1-3}$)amino, qui peut être chaque fois substitué sur l'atome d'azote d'amine par un radical benzoyle ou phénylsulfonyle ou par un radical alkyle en $C_{1-3}$ ou (alkyl en $C_{1-3}$) carbonyle, le cas échéant substitué sur la partie alkyle en $C_{1-3}$ par un radical carboxyle,
représente un radical cycloalkylèneiminocarbonyle ou cycloalkylèneiminosulfonyle ayant 4 à 7 membres, le cas échéant substitué par un radical alkyle en $C_{1-3}$,
représente un radical aminosulfonyle, le cas échéant substitué par un ou deux radicaux alkyle en $C_{1-3}$,
représente un radical phényle, le cas échéant substitué par un atome de fluor, de chlore ou de brome, par un radical trifluorométhyle, aminosulfonyle, alkyle en $C_{1-3}$ ou alcoxy en $C_{1-3}$, lequel peut être substitué en outre, par un autre atome de fluor, de chlore ou de brome ou par un radical trifluorométhyle, alkyle en $C_{1-3}$ ou alcoxy en $C_{1-3}$,
représente un radical alcoxy en $C_{1-3}$, phénylalcoxy en $C_{1-3}$, hétéroaryloxy ou hétéroaryloxyalcoxy en $C_{1-3}$, dont la partie alcoxy peut être chaque fois substituée, en position 2 ou 3, également par un radical amino, (alkyl en $C_{1-3}$)amino, di(alkyl en $C_{1-3}$)amino,
représente un radical cycloalcoxy en $C_{3-7}$, où le radical méthylène en position 3 ou 4 dans un radical cycloalcoxy en $C_{5-7}$, peut être remplacé par un radical NH, où le radical NH peut être substitué par un radical alkyle en $C_{1-3}$, qui peut être substitué en position 2 ou 3 par un radical amino, (alkyl en $C_{1-3}$)amino, di(alkyl en $C_{1-3}$) amino, par un radical (alkyl en $C_{1-3}$)carbonyle, arylcarbonyle ou arylsulfonyle, ou
par un radical aminocarbonyle, (alkyl en $C_{1-3}$)aminocarbonyle ou di (alkyl en $C_{1-3}$)aminocarbonyle, dans lesquels l'atome d'oxygène du radical carbonyle peut être chaque fois remplacé par un radical imino ;
$R_2$ représente l'atome d'hydrogène, de fluor, de chlore ou de brome, un radical alkyle en $C_{1-3}$, hydroxyle ou alcoxy en $C_{1-3}$ ;
$R_3$ représente l'atome d'hydrogène ou un radical alkyle en $C_{1-3}$ ;

$R_4$ représente l'atome d'hydrogène ou un radical alkyle en $C_{1-3}$, le cas échéant substitué par un radical carboxyle, et
$R_5$ représente un radical cyano ou un radical amidino le cas échéant substitué par un ou deux radicaux alkyle en $C_{1-3}$ ;
où par radical hétéroaryle indiqué ci-dessus, on entend un radical hétéroaryle à 5 membres, le cas échéant substitué par un radical alkyle en $C_{1-3}$, qui contient dans la partie hétéroaromatique,
un radical imino le cas échéant substitué par un radical alkyle en $C_{1-3}$, un atome d'oxygène ou de soufre,
un radical imino le cas échéant substitué par un radical alkyle en $C_{1-3}$, et un atome d'oxygène, de soufre ou d'azote,
un radical imino le cas échéant substitué par un radical alkyle en $C_{1-3}$, et deux atomes d'azote, ou
un atome d'oxygène ou un atome de soufre et deux atomes d'azote,
ou un radical hétéroarylène à 6 membres, le cas échéant substitué par un radical alkyle en $C_{1-3}$, qui contient dans la partie hétéroaromatique,
un ou deux atomes d'azote,
les restes carboxyle indiqués dans la définition des restes mentionnés ci-dessus peuvent être remplacés par un radical convertible en un radical carboxy in vivo ou par un radical chargé négativement dans les conditions physiologiques, et
les restes amino et imino indiqués dans la définition des restes mentionnés ci-dessus, peuvent être substitué par un reste clivable in vivo, où
par radical convertible en un radical carboxy in vivo, on entend par exemple, un radical hydroxyméthyle, un radical carboxy estérifié avec un alcool, dans lequel la partie alcoolique est de préférence, un alcanol en $C_1$-$_6$, un phénylalcanol en $C_{1-3}$, un cycloalcanol en $C_{3-9}$, où un cycloalcanol en $C_{5-8}$ peut en outre, être substitué par un ou deux radicaux alkyle en $C_{1-3}$, un cycloalcanol en $C_{5-8}$, dans lequel un radical méthylène en position 3 ou 4 peut être remplacé par un atome d'oxygène ou par un radical imino le cas échéant substitué par un radical alkyle en $C_{1-3}$, phénylalkyle en $C_{1-3}$, phényl (alcoxy en $C_{1-3}$)carbonyle ou alcanoyle en $C_{2-6}$, et la partie cycloalcanol peut être en outre substituée par un ou deux radicaux alkyle en $C_{1-3}$, un cycloalcènol en $C_{4-7}$, un alcènol en $C_{3-5}$, un phénylalcènol en $C_{3-5}$, un alcynol en $C_{3-5}$ ou un phénylalcynol en $C_{3-5}$, avec la condition que aucune liaison ne part d'un atome d'oxygène sur un atome de carbone, qui porte une double ou triple liaison, un (cycloalkyle en $C_{3-8}$)alcanol en $C_{1-3}$, un bicycloalcanol avec au total 8 à 10 atomes de carbone, qui peut être substitué dans la partie bicycloalkyle par un ou deux radicaux alkyle en $C_{1-3}$, un 1,3-dihydro-3-oxo-1-isobenzofurannol ou un alcool de la formule :

$$R_a\text{-CO-O-}(R_bCR_c)\text{-OH}$$

dans laquelle:

$R_a$ représente un radical alkyle en $C_{1-8}$, cycloalkyle en $C_{5-7}$, phényle ou phénylalkyle en $C_{1-3}$,
$R_b$ représente l'atome d'hydrogène, un radical alkyle en $C_{1-3}$, cycloalkyle en $C_{5-7}$ où phényle ;
$R_c$ représente l'atome d'hydrogène ou un radical alkyle en $C_{1-3}$ ;
par radical chargé négativement dans les conditions physiologiques, on entend un radical tétrazol-5-yle, phénylcarbonylaminocarbonyle, trifluorométhylcarbonylaminocarbonyle, (alkyl en $C_{1-6}$)sulfonylamino, phénylsulfonylamino, benzylsulfonylamino, trifluorométhylsulfonylamino, (alkyl en $C_{1-6}$)sulfonylaminocarbonyle, phénylsulfonylaminocarbonyle, benzylsulfonylaminocarbonyle ou (perfluoroalkyl en $C_{1-6}$)sulfonylaminocarbonyle, et
par reste clivable in vivo depuis un radical imino ou amino, par exemple un radical hydroxyle, un radical acyle comme un radical benzoyle le cas échéant mono- ou disubstitué par fluor, chlore, brome ou iode, par un radical alkyle en $C_{1-3}$ ou alcoxy en $C_{1-3}$, où les substituants peuvent être identiques ou différents, un radical pyridinoyle ou un radical alcanoyle en $C_{1-16}$, comme le radical formyle, acétyle, propionyle, butanoyle, pentanoyle ou hexanoyle, un radical 3,3,3-trichloropropionyle ou allyloxycarbonyle, un radical (alcoxy en $C_{1-16}$) carbonyle ou (alkyl en $C_{1-16}$)carbonyloxy, dans lequel les atomes d'hydrogène peuvent être remplacés totalement ou partiellement, par des atomes de fluor ou de chlore, comme le radical méthoxycarbonyle, éthoxycarbonyle, propoxycarbonyle, isopropoxycarbonyle, butoxycarbonyle, t-butoxycarbonyle, pentoxycarbonyle, hexoxycarbonyle, octyloxycarbonyle, nonyloxycarbonyle, décyloxycarbonyle, undécyloxycarbonyle, dodécyloxycarbonyle, hexadécyloxycarbonyle, méthylcarbonyloxy, éthylcarbonyloxy, 2,2,2-trichloroéthylcarbonyloxy, propylcarbonyloxy, isopropylcarbonyloxy, butylcarbonyloxy, t-butylcarbonyloxy, pentylcarbonyloxy, hexylcarbonyloxy, octylcarbonyloxy, nonylcarbonyloxy, décylcarbonyloxy, undécylcarbonyloxy, dodécylcarbonyloxy ou hexadécylcarbonyloxy, un radical phényl(alcoxy en

$C_{1-6}$)carbonyle, comme le radical benzyloxycarbonyle, phényléthoxycarbonyle ou phénylpropoxycarbonyle, un radical 3-aminopropionyle, dans lequel le radical amino peut être mono- ou disubstitué par des radicaux alkyle en $C_{1-6}$ ou cycloalkyle en $C_{3-7}$ et les substituants peuvent être identiques ou différents, un radical (alkyl en $C_{1-3}$)sulfonyl(alcoxy en $C_{2-4}$)carbonyle, (alcoxy en $C_{1-3}$) (alcoxy en $C_{2-4}$) (alcoxy en $C_{2-4}$)carbonyle, $R_a$-CO-O-($R_bCR_c$)-O-CO-, (alkyle en $C_{1-6}$)-CO-NH-($R_dCR_e$)-O-CO- ou (alkyle en $C_{1-6}$) -CO-O-($R_dCR_e$)-($R_dCR_e$)-O-CO-, dans lesquels $R_a$ à $R_c$ sont définis comme indiqué ci-dessus, et $R_d$ et $R_e$, qui peuvent être identiques ou différents, représentent l'atome d'hydrogène ou un radical alkyle en $C_{1-3}$,
leurs isomères et leurs sels.

**2.** Composés de la formule générale I selon la revendication 1, dans laquelle :

Ar représente un radical phénylène, le cas échéant substitué par un atome de fluor, de chlore ou de brome, par un radical méthyle, hydroxyle, méthoxy ou benzyloxy, où le radical phénylène peut être substitué par un autre radical méthyle ;
$R_1$ représente un radical phényle, le cas échéant substitué par un atome de fluor, de chlore ou de brome, par un radical trifluorométhyle, aminosulfonyle, alkyle en $C_{1-3}$ ou alcoxy en $C_{1-3}$, lequel peut être substitué en outre, par un autre atome de fluor, de chlore ou de brome ou par un radical trifluorométhyle, alkyle en $C_{1-3}$ ou alcoxy en $C_{1-3}$,
représente un radical méthyle substitué par un radical diméthylamino, pyrrolidino ou imidazolyle, où la partie imidazolyle peut être substituée par un radical méthyle,
représente un radical amino, (alkyl en $C_{1-5}$)amino, cyclopentylamino ou benzylamino, qui peut être substitué sur l'atome d'azote d'amine par un radical carboxy(alkyle en $C_{1-2}$), (alcoxy en $C_{1-3}$)carbonyl (alkyle en $C_{1-2}$), carboxy (alkyl en $C_{1-2}$)carbonyle ou (alcoxy en $C_{1-3}$)carbonyl (alkyle en $C_{1-2}$)carbonyle,
représente un radical benzoylamino ou phénylsulfonylamino,
représente un radical cyclopropyle, qui est substitué en la position 1 par un radical cycloalkylèneiminocarbonyle ayant 5 à 7 membres,
représente un radical pyrolidinocarbonyle, pipéridinocarbonyle, pyrrolidinosulfonyle ou pipéridinosulfonyle, le cas échéant substitué par le radical méthyle,
représente un radical alcoxy en $C_{1-3}$, dans lequel la partie alcoxy peut être substituée chaque fois en position 2 ou 3 par un radical amino, (alkyl en $C_{1-3}$)amino ou di (alkyl en $C_{1-3}$)amino,
représente un radical phényl(alcoxy en $C_{1-3}$) ou pyridinyloxy,
représente un radical cycloalcoxy en $C_{5-7}$, dans lequel le radical méthylène en position 3 ou 4 peut être remplacé par un radical NH, où le radical NH peut être substitué
par un radical alkyle en $C_{1-3}$ ou alcanoyle en $C_{2-3}$,
par un radical (alcanoyle en $C_{2-3}$)carbonyle ou aminocarbonyle, dans lequel l'atome d'oxygène du radical carbonyle est remplacé par un radical imino,
$R_2$ représente l'atome d'hydrogène, de fluor, de chlore ou de brome, le radical méthyle, hydroxyle ou méthoxy ;
$R_3$ représente l'atome d'hydrogène ou le radical méthyle ;
$R_4$ représente l'atome d'hydrogène ou le radical méthyle ou éthyle, le cas échéant substitué par un radical carboxyle ou (alcoxy en $C_{1-3}$)carbonyle, et
$R_5$ représente un radical cyano ou un radical amidino le cas échéant substitué par un radical (alcoxy en $C_{1-6}$) carbonyle ou benzoyle,
leurs isomères et leurs sels.

**3.** Composés de la formule générale I selon la revendication 1, dans laquelle :

Ar représente un radical phénylène, le cas échéant substitué par le radical méthyle, hydroxyle, méthoxy ou benzyloxy ;
$R_1$ représenté un radical phényle, le cas échéant substitué par l'atome de fluor, de chlore ou de brome, par un radical trifluorométhyle, aminosulfonyle, alkyle en $C_{1-3}$ ou alcoxy en $C_{1-3}$, lequel peut être substitué en . outre, par l'atome de fluor, de chlore ou de brome ou par un radical trifluorométhyle, alkyle en $C_{1-3}$ ou alcoxy en $C_{1-3}$,
représente un radical cyclopropyle, qui est substitué en la position 1 par un radical. cycloalkylèneiminocarbonyle ayant 5 à 7 membres, ou un radical cycloalkylèneiminocarbonyle ayant 4 à 7 membres,
représente un radical pyrolidinocarbonyle, pipéridinocarbonyle ou pyrrolidinosulfonyle, le cas échéant substitué par le radical méthyle ;
$R_2$ représente l'atome d'hydrogène, de fluor, de chlore ou de brome ou le radical méthyle ;

$R_3$ représente l'atome d'hydrogène ou le radical méthyle ;
$R_4$ représente l'atome d'hydrogène ou le radical méthyle ou éthyle substitué par le radical carboxyle, méthoxycarbonyle ou éthoxycarbonyle, et
$R_5$ représente un radical amidino le cas échéant substitué par un radical (alcoxy en $C_{1-6}$)carbonyle ou benzoyle,
leurs isomères et leurs sels.

**4.** Composés suivants de la formule I selon la revendication 1 :

(a) le 2-(5-carbamimidoyl-2-hydroxyphényl)-N-[3-méthyl-4-(pyrrolidin-1-ylcarbonyl)phényl]acétamide,
(b) le 2-(2-benzyloxy-5-carbamimidoylphényl)-N-(2-éthoxycarbonyléthyl)-N-[3-méthyl-4-(pyrrolidin-1-ylcarbonyl)phényl]acétamide,
(c) le 2-(2-hydroxy-5-carbamimidoyl-phényl)-N-(2-éthoxycarbonyléthyl)-N-[3-méthyl-4-(pyrrolidin-1-ylcarbonyl)phényl]acétamide,
(d) le 2-(2-hydroxy-5-carbamimidoyl-phényl)-N-(2-carboxyéthyl)-N-[3-méthyl-4-(pyrrolidin-1-ylcarbonyl)phényl]acétamide,
(e) le 2-(5-carbamimidoyl-2-hydroxyphényl)-N-[3-méthyl-4-(pipéridin-1-ylcarbonyl)phényl]acétamide, et
(f) le 2-(5-carbamimidoyl-2-hydroxyphényl)-N-[3-méthyl-4-(2-aminosulfonylphényl)phényl]acétamide,

dans lesquels le radical amidino peut en outre, être substitué par un radical (alcoxy en $C_{1-6}$)carbonyle ou benzoyle, et leurs sels.

**5.** Le 2-(5-carbamimidoyl-2-hydroxyphényl)-N-[3-méthyl-4-(pyrrolidin-1-ylcarbonyl)phényl]acétamide, et ses sels.

**6.** Sels physiologiquement compatibles des composés selon les revendications 1 à 5, dans lesquels $R_5$ représente l'un des radicaux amidino indiqués aux revendications 1 à 5.

**7.** Agent pharmaceutique, contenant un composé selon au moins l'une des revendications 1 à 5, dans lequel $R_5$ représente l'un des radicaux amidino indiqués aux revendications 1 à 5, ou un sel selon la revendication 6, avec le cas échéant, un ou plusieurs supports et/ou agents de dilution inertes.

**8.** Utilisation d'un composé selon au moins l'une des revendications 1 à 5, dans lequel $R_5$ représente l'un des radicaux amidino indiqués aux revendications 1 à 5, ou d'un sel selon la revendication 6, pour la préparation d'un agent pharmaceutique avec action antithrombotique.

**9.** Procédé de préparation d'un agent pharmaceutique selon la revendication 7, **caractérisé en ce que** l'on incorpore de manière non chimique, un composé selon au moins l'une des revendications 1 à 5, dans lequel $R_5$ représente l'un des radicaux amidino indiqués aux revendications 1 à 5, ou un sel selon la revendication 6, dans un ou plusieurs supports et/ou agents de dilution inertes.

**10.** Procédé de préparation des composés selon les revendications 1 à 6, **caractérisé en ce que**

a) on acyle un composé de la formule générale :

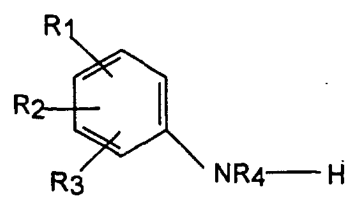

(II)

dans laquelle $R_1$ à $R_4$ sont définis comme indiqué aux revendications 1 à 5, avec un acide carboxylique de la formule générale :

$$HO—CO—CH_2—Ar—R_5 \quad (III)$$

dans laquelle Ar et $R_5$ sont définis comme indiqué aux revendications 1 à 5, ou avec son dérivé réactif, ou b) pour la préparation d'un composé de la formule générale I, dans laquelle $R_5$ représente un radical amidino, qui peut être substitué par un ou deux radicaux alkyle en $C_{1-3}$, on fait réagir un composé le cas échéant formé dans le mélange réactionnel de la formule générale :

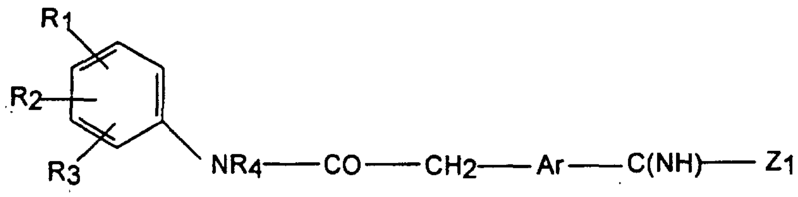

(IV)

dans laquelle $R_1$ à $R_4$ et Ar sont définis comme indiqué aux revendications 1 à 5, et

$Z_1$ représente un radical alcoxy, aralcoxy, alkylthio ou aralkylthio,

avec une amine de la formule générale :

$$H - R_6NR_7 \quad (V)$$

dans laquelle $R_6$ et $R_7$, qui peuvent être identiques ou différents, signifient chaque fois, l'atome d'hydrogène ou un radical alkyle en $C_{1-3}$, ou avec un de ses sels, et

si souhaité, on convertit un composé ainsi obtenu de la formule générale I, qui contient un radical amino ou imino, à l'aide d'un dérivé acyle approprié, en un composé acyle correspondant de la formule générale I, et/ou

on convertit un composé ainsi obtenu de la formule générale I, qui contient un radical carboxy estérifié, par hydrolyse en l'acide carboxylique correspondant de la formule générale I, et/ou

un composé ainsi obtenu de la formule générale I, qui contient un radical carboxy, est converti par estérification en un ester correspondant, et/ou

un reste protecteur utilisé pendant les réactions pour protéger des radicaux réactifs est clivé et/ou

un composé ainsi obtenu de la formule générale I est séparé en ses stéréoisomères, et/ou

un composé ainsi obtenu de la formule générale I est converti en ses sels, en particulier pour l'utilisation pharmaceutique, en ses sels physiologiquement compatibles avec un acide ou une base inorganique ou organique.